# EUROPEAN PATENT APPLICATION

(11) **EP 4 140 489 A1**
(43) Date of publication of application: **01.03.2023**
(21) Application number: 22199066.6
(22) Date of filing: 07.02.2019
(51) Int. Cl.: A61K 31/565, A61P 15/18, A61K 9/00

(54) **CONTRACEPTIVE COMPOSITION WITH REDUCED CARDIOVASCULAR EFFECTS**

(30) Priority: 07.02.2018 EP 18155571; 07.03.2018 EP 18160586
(62) Divisional of application: 19703103.2
(71) Applicant: Estetra SRL, 4000 Liège (BE)
(72) Inventor: JOST, Maud, 4020 Jupille-sur-Meuse (BE); RAUSIN, Glwadys, 4870 Trooz (BE)
(74) Representative: De Clercq & Partners

(57) **Abstract**

The present invention relates to a contraceptive method with reduced cardiovascular effects, such as reduced thromboembolism risk, such as reduced venous thromboembolism (VTE) risk and reduced aortic thromboembolism (ATE) risk. The method of the invention comprises administering to a female mammal an effective amount of an estetrol component in combination with a progestogenic component. The method enjoys a favourable profile for thromboembolism compared to currently available methods which employs contraceptives from the so-called second, third or fourth generation.

## Description

### Field of the invention

The present invention relates to a contraceptive method with reduced cardiovascular effects, such as reduced thromboembolism risk, such as reduced venous thromboembolism (VTE) risk and reduced aortic thromboembolism (ATE) risk.

The method of the invention comprises administering to a female mammal an effective amount of an estetrol component in combination with a progestogenic component.

As further detailed herein, the method enjoys a favourable profile for thromboembolism compared to currently available methods which employs contraceptives from the so-called second, third or fourth generation.

### Background art

The first Combined Hormonal Contraceptives (CHCs) contained a dose of estrogen of over 50µg. However, studies soon showed that these preparations were associated with an unacceptable increase in risk of cardiovascular effects and that this was dependent on the dose of the estrogen component. Subsequent studies showed that the risk was much reduced by lowering the dose of estrogen and this resulted in the introduction of newer preparations containing <50 µg estrogen. However, with the lower doses of estrogen came the realisation that the characteristics of the progestogen may also have an influence on the risk of thromboembolism.

The early progestogens were all derived from testosterone and concerns over their possible cardiovascular risk resulted in the evolution of a newer set of progestogens which were designed with the intention of reducing their adverse cardiovascular impact. As a result of their phased introduction to the market, the different types of CHCs have been categorised into 'generations':
- first generation (estrones): refers to the original CHCs that contained estrogens other than ethinylestradiol (EE), and progestogens such as norethisterone and norethindrone;
- second generation: (gonanes): refers to the products developed in the 1970s that combined ethinylestradiol with progestogens derived from testosterone (most commonly levonorgestrel (LNG) and norgestrel). These had a higher affinity for the progesterone and androgen receptors compared with the first generation CHCs;
- third generation: refers to products developed in the 1980s containing progestogens derived from levonorgestrel (gestodene, desogestrel and norgestimate). It was anticipated these would have less metabolic side effects;
- fourth generation: refers to products containing the anti-androgenic progestogens, typically cyproterone acetate, drospirenone (DRSP), dienogest and chlormadinone.

It is important to note that fourth generation COCs have been developed to offer better Quality of Life. The COCs using for example DRSP indeed display an attenuation of a number of side effects compared to earlier generations COCs.

Even though VTE is a rare side effect of all Combined Oral Contraceptives (COCs), it has been shown that:
- women using a 3^{rd} generation COC with 30µg of ethinylestradiol have a small increased risk of VTE compared to women using 2^{nd} generation COCs;
- for 3^{rd} generation COCs with 20µg of ethinylestradiol the epidemiological data do not suggest a lower VTE risk than for those containing 30µg of ethinylestradiol;
- there is an excess risk of VTE during the first year a woman ever uses any COC.

The impact of the relative risk of VTE of 3^{rd} generation compared to 2^{nd} generation COCs on the number of additional cases would therefore be greatest in the first year a woman ever uses a COC. This should be taken into account when a COC is prescribed for and used by a woman for the first time.

The estimated risk of blood clot occurrence in users of a CHC based on LNG, norethisterone or norgestimate is from 5 to 7 per 10,000 women during one year. For users of a CHC that contains drospirenone, the approximate risk of blood clot occurrence is from 9 to 12 per 10,000 women during one year.

By comparison, the estimated risk of blood clot occurrence in non-CHC users who are not pregnant is around 2 per 10,000 women during one year.

As such, the use of combined contraceptives have been associated with an increased risk in venous thromboembolic events (VTEs). In comparison with non-users, it is generally accepted that the use of second generation COCs multiply by 2 the risk of VTE and that the use of 3^{rd} and 4^{th} generation COCs multiply the risk by 4. The absolute risk of VTE associated with the use of a specific combined contraceptive can only be assessed during very large epidemiological trials. However, and as requested by the European Medicinal Agency, several surrogate markers of the VTE risk can be measured in smaller clinical settings to estimate the risk.

Another way to present the evolution of the venous thrombosis risk as a function of the generation of the COC used is by looking at the relative risk when compared to no oral contraceptive used (relative risk of 1.0). A number of publications (WHO (1995) Lancet 346, 1575-1588; Jick et al. (1995) Lancet 346, 1589-1593; Spitzer et al. (1996) BMJ 312, 127-132; Vlieg et al. (2009) BMJ 339:b2921; Lidegaard et al. (2009) BMJ 339:b2890; Lidegaard et al. (2011) BMJ 343:d6423) have addressed this issue and their findings are summarized in the Table below.

| | Relative Risk |
|---|---|
| No COC | 1.0 |
| 2nd generation COC (levonorgestrel) | 3.2 - 3.5 |
| 3rd generation COC (desogestrel/gestodene) | 4.8 - 9.1 |
| 4th generation COC (drospirenone) | 4.0 - 6.3 |

All the approaches described above relied on COCs employing synthetic estrogens such as ethinyl estradiol (EE), however.

Of particular importance is the fact that estrogens participate in the regulation of the synthesis of a variety of proteins in the liver, such as angiotensinogen, Sex Hormone Binding Globulin (SHBG), ceruloplasmin, Corticosteroid Binding Globulin (CBG), some coagulation factors, coagulation inhibitors or fibrinolysis markers. Changes in these haemostasis markers under the influence of strong estrogens such as EE may collectively contribute to create an imbalance between pro-coagulation and anti-coagulation factors which can enhance the risks of Venous ThromboEmbolism (VTE) events.

SHBG plasma levels are a reliable marker of the influence of an estrogen on the synthesis of these proteins by liver cells. This means that a correlation could exist between the level of SHBG induced by a specific COC and the risk of VTE associated with that COC (Odlind V, et al.; Acta Obstet Gynecol Scand 2002; 81:482).

Although cohort studies performed on a sufficient number of subjects are required to evaluate the risk of VTE with a specific COC, different haemostatic markers and carrier proteins (such as SHBG) can be measured to estimate this risk on a limited number of subjects.

On the effect of the progestogenic component, a substantial number of studies plus two good meta-analyses have now evaluated the thrombotic risk with drospirenone containing CHCs. These use a number of different data sources across different countries and, with the exception the study by Dinger (Dinger J, Assmann A, Möhner S, Minh TD. Risk of venous thromboembolism and the use of dienogest- and drospirenone-containing oral contraceptives: results from a German case-control study. J Fam Plann Reprod Health Care. 2010; 36(3):123-9), all consistently show an elevated risk of VTE in drospirenone users relative to levonorgestrel users that was, in most cases, statistically significant. The risk estimates most commonly range between about 1.5 and 2 times versus levonorgestrel. While limitations can always be identified for observational studies, bias and residual confounding are unlikely to account for the entire risk increase that is observed. The Sidney study (Sidney S, Cheetham TC, Connell FA, Ouellet-Hellstrom R, Graham DJ, Davis D, Sorel M, Quesenberry CP Jr, Cooper WO. Recent combined hormonal contraceptives (CHCs) and the risk of thromboembolism and other cardiovascular events in new users. Contraception 2013, 87(1): 93-100) in particular is considered to provide strong evidence as this analysis was restricted to new users (of which there were almost 140,000 in this cohort study).

Overall, consistent findings support an excess VTE risk with DRSP in relation to LNG.

In a recently published study, Kluft et al. report the observation of reduced haemostatic effects with COCs using estetrol as the estrogen by comparison with a COC using EE as the estrogen (Kluft et al., Reduced hemostatic effects with drospirenone-based oral contraceptives containing estetrol vs. ethinyl estradiol, Contraception, 95 (2017), p.140-147).

In this publication, both the new estetrol-based COC and the comparator (YAZ^{®} commercial product) used drospirenone as the progestogen. The conclusion that can be drawn from this study is that the estrogen estetrol offers a safer haemostatic profile than the estrogen ethinyl estradiol, when both are associated with drospirenone.

Regarding the effect of the progestogen on the safety profile, as discussed above and reported by Kluft et al. themselves (page 141, left-hand column, beginning of 4^{th} paragraph), *"markers and variables are notably less modified with LNG compared to [*...*] DRSP."*

For this reason, and even though the Kluft at al. publication teaches a better safety profile when EE is replaced by estetrol in COCs using DRSP as the progestogen, it was unexpected before the present invention that a COC using Estetrol and DRSP would compare favourably to a 2^{nd} generation COC, such as one using LNG as the progestogen. In particular, the Kluft at al. publication does not disclose nor suggest that a composition comprising an estetrol component and DRSP as the progestogenic component reduces the blood clots risk to a level which is lower than the lower limit for blood clots risk associated with DRSP-based COCs. In view of the common general knowledge that a fourth generation COC (DRSP-based), despite having a number of advantages in terms of reduced side-effects, presents a much higher blood clots risk than an earlier generation COC, it was not conceivable before the present invention that a DRSP-based COC could present a blood clots risk outside the range of the risk associated with DRSP-based COCs of the prior art.

WO2018/024912 and WO2018/065076 disclose the use of estetrol to alleviate the symptoms of dysmenorrhea. These patent applications are silent about the reduction of blood clot risk. Table 7 in these patent applications present the same clinical data as Table 1 of the Kluft at al. publication, although in a slightly different manner: while the Kluft at al. publication presents median values (expressed as percentage of the baseline value, taken as 100%), the patent applications present mean values (percentage changes).

There thus remains a need for a contraceptive approach which provides a better safety profile than currently available COCs, and in particular which displays a lower risk of thromboembolic events. This is especially critical in at-risk populations, such as first-ever users; switchers/re-starters with a break of >4 weeks; and women with increased baseline risk for VTE due to one or more major risk factors (such as, but not limited to, BMI>30, older age, and positive personal and/or family history).

### Summary of the invention

The present invention relates to a contraceptive method with reduced cardiovascular effects, comprising administering to a female mammal an effective amount of an estetrol component in combination with a progestogenic component.

In one embodiment of the invention, the number, frequency and/or severity of VTE events is reduced, compared to other contraceptive methods.

In a particular embodiment, the risk of VTE during the contraceptive method of the invention is similar to the VTE risk during use of a CHC based on levonorgestrel, norgestimate or norethisterone.

In a preferred embodiment, the risk of VTE during the contraceptive method of the invention is lower than the VTE risk during use of a CHC based on levonorgestrel, norgestimate or norethisterone.

In one embodiment of the invention, the number, frequency and/or severity of pulmonary embolism events is reduced, compared to other contraceptive methods.

In another embodiment of the invention, the number, frequency and/or severity of deep venous thrombosis (DVT) events is reduced, compared to other contraceptive methods.

In another embodiment of the invention, no haemostatic change that exceeds the boundaries of the normal range, as further defined herein, occurs upon administration of the compositions of the invention.

In yet another embodiment, the method of the invention displays smaller "haemostatic changes" (as further defined herein) than a contraceptive method employing a 2^{nd}, 3^{rd} or 4^{th} generation CHC. In a particularly preferred embodiment, the invention displays smaller "haemostatic changes" than a contraceptive method employing a 2^{nd} generation CHC.

In a further embodiment, markers of coagulation and/or fibrinolysis markers display changes (by comparison with their levels before administration of any contraceptive composition) during the contraceptive method of the invention which are smaller than the changes observed with another contraceptive method.

In a particular embodiment, markers of coagulation and/or fibrinolysis markers display smaller changes during the contraceptive method of the invention than during a contraceptive method using a 2^{nd}, 3^{rd} or 4^{th} generation CHC. In a preferred embodiment, markers of coagulation and/or fibrinolysis markers display smaller changes during the contraceptive method of the invention than during a contraceptive method using a 2^{nd} generation CHC.

In one embodiment of the invention, an effective amount of an estetrol component is used in a combined oral contraceptive composition which uses drospirenone as the progestogenic component, in order to reduce the risk of blood clots associated with the use of drospirenone.

In another embodiment of the invention, an effective amount of an estetrol component is used in a combined oral contraceptive composition which uses drospirenone as the progestogenic component, in order to reduce the risk of blood clots associated with the use of a combined oral contraceptive composition which includes drospirenone.

In all these aforementioned embodiments, the risk of blood clots is advantageously reduced to less than 8, preferably less than 7, more preferably less than 6, even more preferably less than 5, yet even more preferably less than 4 per 10,000 women using said contraceptive during one year.

In one embodiment of the invention, the method involves the administration of an effective amount of an estetrol component and of a progestogenic component.

In some embodiments of the invention, the estetrol and the progestogenic components are included in a single dosage unit. In further embodiments, the dosage unit is a daily dosage unit.

In further embodiments, the progestogenic component is drospirenone and that component is used at a daily dose of from 0.5 mg to 10 mg, preferably at a daily dose of from 1 mg to 4 mg.

In yet further embodiments, the estetrol component is used at a daily dose of from 1 mg to 40 mg, preferably at a daily dose of from 5 mg to 25 mg, even more preferably at a daily dose of from 10 mg to 20 mg. In particular embodiments, the estetrol component is estetrol monohydrate.

In a specific embodiment of the invention, the estetrol component is estetrol monohydrate at a daily dose of about 15 mg and the progestogenic component is drospirenone at a daily dose of about 3 mg.

In the following numbered paragraphs 1 to 22, additional embodiments of the invention are described.
1. A composition for use in a contraceptive method in a female mammal, wherein said composition comprises an effective amount of an estetrol component.
2. The composition for use of paragraph 1 wherein said composition further comprises a progestogenic component.
3. The composition for use of paragraph 1 or 2 wherein said method results in the reduction of one or more of the number, the frequency and the severity of VTE events compared to other contraceptive methods.
4. The composition for use of paragraph 3 wherein said progestogenic component is drospirenone.
5. A composition for use according to any one of paragraphs 1-4 wherein the estetrol component is used at a daily dose of from 1 mg to 40 mg, preferably at a daily dose of from 5 mg to 25 mg.
6. The composition for use of paragraph 4 wherein drospirenone is used at a daily dose of from 0.5 mg to 10 mg, preferably at a daily dose of from 1 mg to 4 mg.
7. A composition for use according to any one of paragraphs 1-6 wherein the administration method is a combined method with an administration-free interval of about 7 days, preferably with an administration-free interval of about 4 days.
8. A composition for use according to any one of paragraphs 1-7 wherein the estetrol component is estetrol, preferably estetrol monohydrate.
9. The composition for use according to paragraph 8 wherein the estetrol component is used at a daily dose of about 15 mg of estetrol.
10. The composition for use according to paragraph 9 wherein the drospirenone is used at a daily dose of about 3 mg.
11. A composition for use according to any of the preceding paragraphs wherein the composition is formulated as an oral dosage unit.
12. The composition for use according to paragraph 11 wherein the oral dosage unit is formulated to correspond to a daily dosage unit.
13. A composition for use according to any one of paragraphs 3-12 wherein the risk of blood clots occurrence is less than 12, preferably less than 11, more preferably less than 10, even more preferably less than 9 per 10,000 women using said composition during one year.
14. A composition for use according to paragraph 13 wherein the risk of blood clots occurrence is less than 8, preferably less than 7, more preferably less than 6, even more preferably less than 5, yet even more preferably less than 4 per 10,000 women using said composition during one year.
15. A composition for use according to any one of the paragraphs 1-12 wherein the number of VTE cases is less than 15-20 VTE per 10,000 women during one year.
16. A composition for use according to paragraph 15 wherein the number of VTE cases is less than 15, preferably less than 13, more preferably less than 11, even more preferably less than 10 VTE per 10,000 women during one year.
17. A composition for use according to any one of the preceding paragraphs wherein the VTE risk is lower than the VTE risk associated with a 3^{rd} generation or with a 4^{th} generation COC.
18. A composition for use according to any one of the preceding paragraphs wherein the VTE risk is lower than the VTE risk associated with a 2^{nd} generation COC.
19. A composition for use according to paragraph 17 or 18 wherein the profile of side effects other than VTE is better than the profile of side effects associated with a 2^{nd} generation COC.
20. A composition for use according to any one of the preceding paragraphs wherein the relative venous thrombosis risk, by comparison to a woman not using COCs, is lower than 4.5, preferably lower than 4.0, more preferably lower than 3.5, even more preferably lower than 3.2, yet even more preferably lower than 3.0.
21. A composition for use according to any one of the preceding paragraphs wherein the percentage of subjects in the high risk category for thrombosis, defined as subjects having an odds ratio of 2 or above for thrombosis risk compared to subjects not using COCs, represents less than 40%, preferably less than 35%, more preferably less than 30%, even more preferably less than 25%, yet even more preferably less than 20%, of the total population using said composition.
22. A composition for use according to any one of the preceding paragraphs wherein the composition is administered to a woman who is a first-ever user, or a woman who is a switcher or a re-starter with a break of >4 weeks, or a woman with increased baseline risk for VTE due to one or more major risk factors selected from BMI>30, older age, and positive personal and/or family history.

In the following numbered paragraphs 31 to 52, additional embodiments of the invention are described.
31. A contraceptive method for a female mammal, which comprises administration of a composition comprising an effective amount of an estetrol component.
32. The contraceptive method of paragraph 31 wherein said composition further comprises a progestogenic component.
33. The contraceptive method of paragraph 31 or 32 wherein said method results in the reduction of one or more of the number, the frequency and the severity of VTE events compared to other contraceptive methods.
34. The contraceptive method of paragraph 33 wherein said progestogenic component is drospirenone.
35. A contraceptive method according to any one of paragraphs 31-34 wherein the estetrol component is used at a daily dose of from 1 mg to 40 mg, preferably at a daily dose of from 5 mg to 25 mg.
36. The contraceptive method of paragraph 34 wherein drospirenone is used at a daily dose of from 0.5 mg to 10 mg, preferably at a daily dose of from 1 mg to 4 mg.
37. A contraceptive method according to any one of paragraphs 31-36 wherein the administration method is a combined method with an administration-free interval of about 7 days, preferably with an administration-free interval of about 4 days.
38. A contraceptive method according to any one of paragraphs 31-37 wherein the estetrol component is estetrol, preferably estetrol monohydrate.
39. The contraceptive method according to paragraph 38 wherein the estetrol component is used at a daily dose of about 15 mg of estetrol.
40. The contraceptive method according to paragraph 39 wherein the drospirenone is used at a daily dose of about 3 mg.
41. A contraceptive method according to any of the preceding paragraphs wherein the composition is formulated as an oral dosage unit.
42. The contraceptive method according to paragraph 41 wherein the oral dosage unit is formulated to correspond to a daily dosage unit.
43. A contraceptive method according to any one of paragraphs 33-42 wherein the risk of blood clots occurrence is less than 12, preferably less than 11, more preferably less than 10, even more preferably less than 9 per 10,000 women using said composition during one year.
44. A contraceptive method according to paragraph 43 wherein the risk of blood clots occurrence is less than 8, preferably less than 7, more preferably less than 6, even more preferably less than 5, yet even more preferably less than 4 per 10,000 women using said composition during one year.
45. A contraceptive method according to any one of the paragraphs 31-42 wherein the number of VTE cases is less than 15-20 VTE per 10,000 women during one year.
46. A contraceptive method according to paragraph 45 wherein the number of VTE cases is less than 15, preferably less than 13, more preferably less than 11, even more preferably less than 10 VTE per 10,000 women during one year.
47. A contraceptive method according to any one of the preceding paragraphs wherein the VTE risk is lower than the VTE risk associated with a 3^{rd} generation or with a 4^{th} generation COC.
48. A contraceptive method according to any one of the preceding paragraphs wherein the VTE risk is lower than the VTE risk associated with a 2^{nd} generation COC.
49. A contraceptive method according to paragraph 47 or 48 wherein the profile of side effects other than VTE is better than the profile of side effects associated with a 2^{nd} generation COC.
50. A contraceptive method according to any one of the preceding paragraphs wherein the relative venous thrombosis risk, by comparison to a woman not using COCs, is lower than 4.5, preferably lower than 4.0, more preferably lower than 3.5, even more preferably lower than 3.2, yet even more preferably lower than 3.0.
51. A contraceptive method according to any one of the preceding paragraphs wherein the percentage of subjects in the high risk category for thrombosis, defined as subjects having an odds ratio of 2 or above for thrombosis risk compared to subjects not using COCs, represents less than 40%, preferably less than 35%, more preferably less than 30%, even more preferably less than 25%, yet even more preferably less than 20%, of the total population using said composition.
52. A contraceptive method according to any one of the preceding paragraphs wherein the composition is administered to a woman who is a first-ever user, or a woman who is a switcher or a re-starter with a break of >4 weeks, or a woman with increased baseline risk for VTE due to one or more major risk factors selected from BMI>30, older age, and positive personal and/or family history.

In the following numbered paragraphs 61 to 82, additional embodiments of the invention are described.
61. Use of an effective amount of an estetrol component in the manufacture of a composition for a contraception method in a female mammal.
62. Use of paragraph 61 wherein said composition further comprises a progestogenic component.
63. Use of paragraph 61 or 62 wherein said method results in the reduction of one or more of the number, the frequency and the severity of VTE events compared to other contraceptive methods.
64. Use of paragraph 63 wherein said progestogenic component is drospirenone.
65. Use according to any one of paragraphs 61-64 wherein the estetrol component is used at a daily dose of from 1 mg to 40 mg, preferably at a daily dose of from 5 mg to 25 mg.
66. Use of paragraph 64 wherein drospirenone is used at a daily dose of from 0.5 mg to 10 mg, preferably at a daily dose of from 1 mg to 4 mg.
67. Use according to any one of paragraphs 61-66 wherein the method is a combined method with an administration-free interval of about 7 days, preferably with an administration-free interval of about 4 days.
68. Use according to any one of paragraphs 61-67 wherein the estetrol component is estetrol, preferably estetrol monohydrate.
69. Use according to paragraph 68 wherein the estetrol component is used at a daily dose of about 15 mg of estetrol.
70. Use according to paragraph 69 wherein the drospirenone is used at a daily dose of about 3 mg.
71. Use according to any of the preceding paragraphs wherein the composition is formulated as an oral dosage unit.
72. Use according to paragraph 71 wherein the oral dosage unit is formulated to correspond to a daily dosage unit.
73. Use according to any one of paragraphs 63-72 wherein the risk of blood clots occurrence is less than 12, preferably less than 11, more preferably less than 10, even more preferably less than 9 per 10,000 women using said composition during one year.
74. Use according to paragraph 73 wherein the risk of blood clots occurrence is less than 8, preferably less than 7, more preferably less than 6, even more preferably less than 5, yet even more preferably less than 4 per 10,000 women using said composition during one year.
75. Use according to any one of the paragraphs 61-72 wherein the number of VTE cases is less than 15-20 VTE per 10,000 women during one year.
76. Use according to paragraph 75 wherein the number of VTE cases is less than 15, preferably less than 13, more preferably less than 11, even more preferably less than 10 VTE per 10,000 women during one year.
77. Use according to any one of the preceding paragraphs wherein the VTE risk is lower than the VTE risk associated with a 3^{rd} generation or with a 4^{th} generation COC.
78. Use according to any one of the preceding paragraphs wherein the VTE risk is lower than the VTE risk associated with a 2^{nd} generation COC.
79. Use according to paragraph 77 or 78 wherein the profile of side effects other than VTE is better than the profile of side effects associated with a 2^{nd} generation COC.
80. Use according to any one of the preceding paragraphs wherein the relative venous thrombosis risk, by comparison to a woman not using COCs, is lower than 4.5, preferably lower than 4.0, more preferably lower than 3.5, even more preferably lower than 3.2, yet even more preferably lower than 3.0.
81. Use according to any one of the preceding paragraphs wherein the percentage of subjects in the high risk category for thrombosis, defined as subjects having an odds ratio of 2 or above for thrombosis risk compared to subjects not using COCs, represents less than 40%, preferably less than 35%, more preferably less than 30%, even more preferably less than 25%, yet even more preferably less than 20%, of the total population using said composition.
82. Use according to any one of the preceding paragraphs wherein the composition is administered to a woman who is a first-ever user, or a woman who is a switcher or a re-starter with a break of >4 weeks, or a woman with increased baseline risk for VTE due to one or more major risk factors selected from BMI>30, older age, and positive personal and/or family history.

In the following numbered paragraphs 91 to 105, additional embodiments of the invention are described.
91. A contraceptive method for a female mammal, which comprises administration of a combined oral contraceptive comprising an effective amount of an estetrol component as the estrogenic component and an effective amount of drospirenone as the progestogenic component, wherein the estetrol component reduces the risk of thromboembolism associated with the use of drospirenone.
92. The contraceptive method of paragraph 91, wherein said risk of thromboembolism is reduced to less than 8, preferably less than 7, more preferably less than 6, even more preferably less than 5, yet even more preferably less than 4 per 10,000 women using said contraceptive during one year.
93. The contraceptive method of paragraphs 91 or 92, wherein the estetrol component is used at a daily dose of from 1 mg to 40 mg, preferably at a daily dose of from 5 mg to 25 mg.
94. The contraceptive method of any of paragraphs 91-93 wherein drospirenone is used at a daily dose of from 0.5 mg to 10 mg, preferably at a daily dose of from 1 mg to 4 mg.
95. The contraceptive method of any one of paragraphs 91-94 wherein the administration method is a combined method with an administration-free interval of about 7 days, preferably with an administration-free interval of about 4 days.
96. The contraceptive method of any one of paragraphs 91-95 wherein the estetrol component is estetrol, preferably estetrol monohydrate.
97. The contraceptive method of paragraph 96 wherein estetrol is used at a daily dose of about 15 mg of estetrol.
98. The contraceptive method of paragraph 97 wherein drospirenone is used at a daily dose of about 3 mg.
99. The contraceptive method of any of the preceding paragraphs wherein the contraceptive is formulated as an oral dosage unit.
100.The contraceptive method according to paragraph 99 wherein the oral dosage unit is formulated to correspond to a daily dosage unit.
101 .The contraceptive method according to any one of the preceding paragraphs wherein the VTE risk is lower than the VTE risk associated with a 2^{nd} generation COC.
102.The contraceptive method according to paragraphs 100 or 101 wherein the profile of side effects other than VTE is better than the profile of side effects associated with a 2^{nd} generation COC.
103.The contraceptive method according to any one of the preceding paragraphs wherein the relative venous thrombosis risk, by comparison to a woman not using COCs, is lower than 3.5, preferably lower than 3.2, more preferably lower than 3.0, even more preferably lower than 2.5, yet even more preferably lower than 2.0.
104.The contraceptive method according to any one of the preceding paragraphs wherein the percentage of subjects in the high risk category for thrombosis, defined as subjects having an odds ratio of 2 or above for thrombosis risk compared to subjects not using COCs, represents less than 40%, preferably less than 35%, more preferably less than 30%, even more preferably less than 25%, yet even more preferably less than 20%, of the total population using said composition.
105.The contraceptive method according to any one of the preceding paragraphs wherein the composition is administered to a woman who is a first-ever user, or a woman who is a switcher or a re-starter with a break of >4 weeks, or a woman with increased baseline risk for VTE due to one or more major risk factors selected from BMI>30, older age, and positive personal and/or family history.

In the following numbered paragraphs 111 to 125, additional embodiments of the invention are described.
111.Use of an effective amount of an estetrol component as the estrogenic component in the manufacture of a combined oral contraceptive for a contraception method in a female mammal wherein an effective amount of drospirenone is the progestogenic component, and wherein the estetrol component reduces the risk of thromboembolism associated with the use of drospirenone.
112.Use of paragraph 111 wherein said risk of thromboembolism is reduced to less than 8, preferably less than 7, more preferably less than 6, even more preferably less than 5, yet even more preferably less than 4 per 10,000 women using said contraceptive during one year.
113.Use of paragraphs 111 or 112 wherein the estetrol component is used at a daily dose of from 1 mg to 40 mg, preferably at a daily dose of from 5 mg to 25 mg.
114.Use of any of paragraphs 111-113 wherein drospirenone is used at a daily dose of from 0.5 mg to 10 mg, preferably at a daily dose of from 1 mg to 4 mg.
115.Use of any one of paragraphs 111-114 wherein the administration method is a combined method with an administration-free interval of about 7 days, preferably with an administration-free interval of about 4 days.
116.Use of any one of paragraphs 111-115 wherein the estetrol component is estetrol, preferably estetrol monohydrate.
117.Use of paragraph 116 wherein estetrol is used at a daily dose of about 15 mg of estetrol.
118.Use of paragraph 117 wherein drospirenone is used at a daily dose of about 3 mg.
119.Use of any of the preceding paragraphs wherein the contraceptive is formulated as an oral dosage unit.
120.Use according to paragraph 119 wherein the oral dosage unit is formulated to correspond to a daily dosage unit.
121.Use according to any one of the preceding paragraphs wherein the VTE risk is lower than the VTE risk associated with a 2^{nd} generation COC.
122.Use according to paragraphs 120 or 121 wherein the profile of side effects other than VTE is better than the profile of side effects associated with a 2^{nd} generation COC.
123.Use according to any one of the preceding paragraphs wherein the relative venous thrombosis risk, by comparison to a woman not using COCs, is lower than 3.5, preferably lower than 3.2, more preferably lower than 3.0, even more preferably lower than 2.5, yet even more preferably lower than 2.0.
124.Use according to any one of the preceding paragraphs wherein the percentage of subjects in the high risk category for thrombosis, defined as subjects having an odds ratio of 2 or above for thrombosis risk compared to subjects not using COCs, represents less than 40%, preferably less than 35%, more preferably less than 30%, even more preferably less than 25%, yet even more preferably less than 20%, of the total population using said composition.
125.Use according to any one of the preceding paragraphs wherein the composition is administered to a woman who is a first-ever user, or a woman who is a switcher or a re-starter with a break of >4 weeks, or a woman with increased baseline risk for VTE due to one or more major risk factors selected from BMI>30, older age, and positive personal and/or family history.

The present method employs an estetrol component which is a natural estrogen (i.e. found in nature) and a biogenic estrogen (i.e. occurring naturally in the human body).

Because biogenic estrogens are naturally present in the fetal and female body, a good tolerability and safety profile are observed, particularly if the serum levels resulting from the exogenous administration of such estrogens do not substantially exceed naturally occurring concentrations.

### Description of the Figures

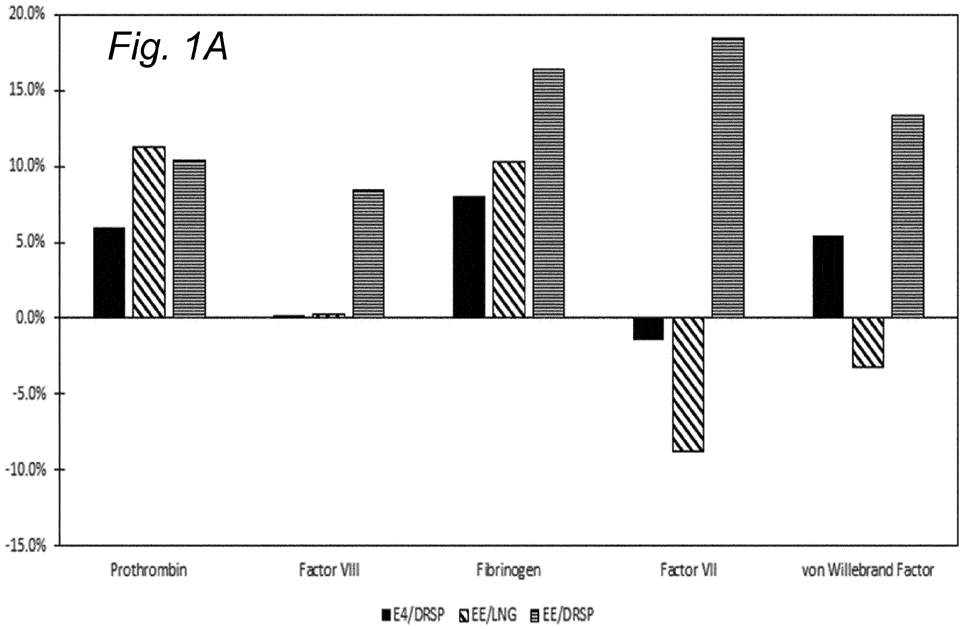

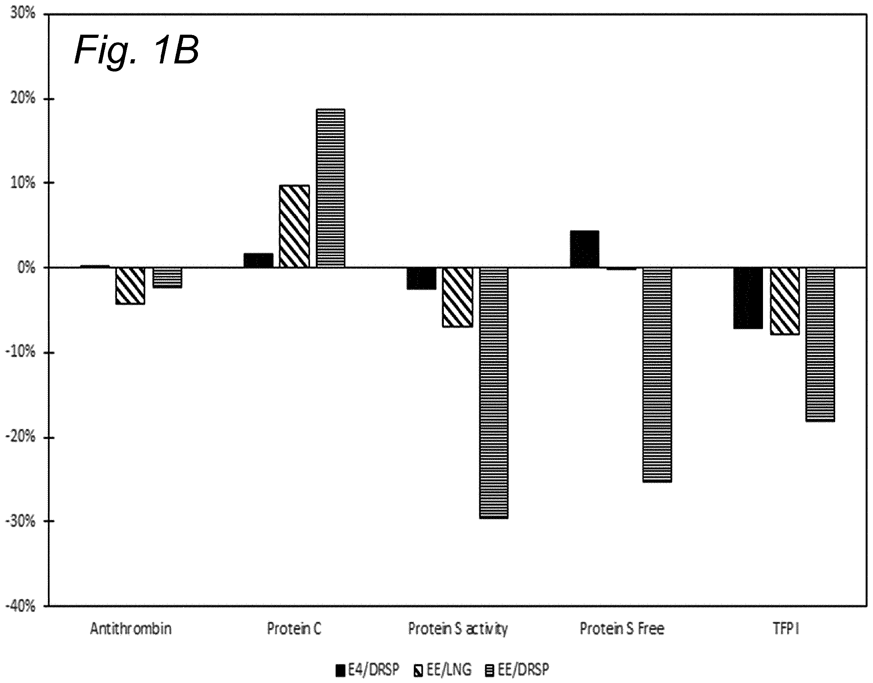

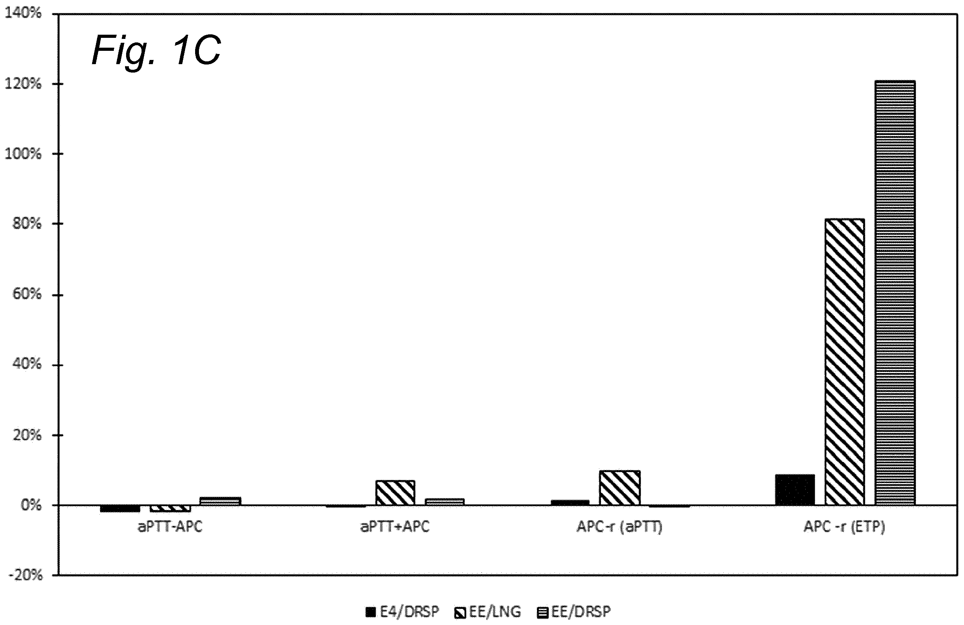

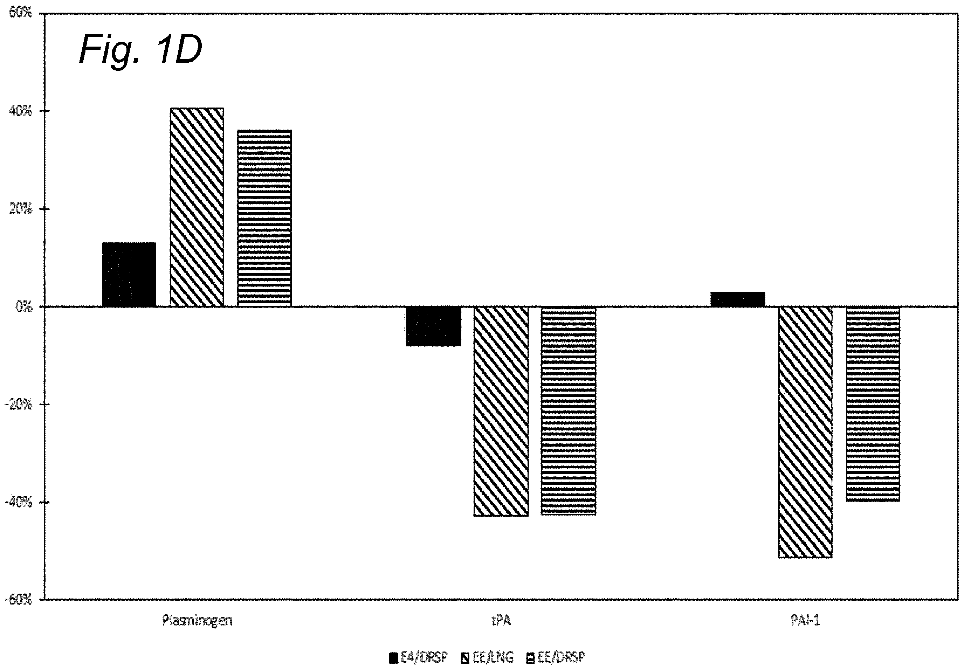

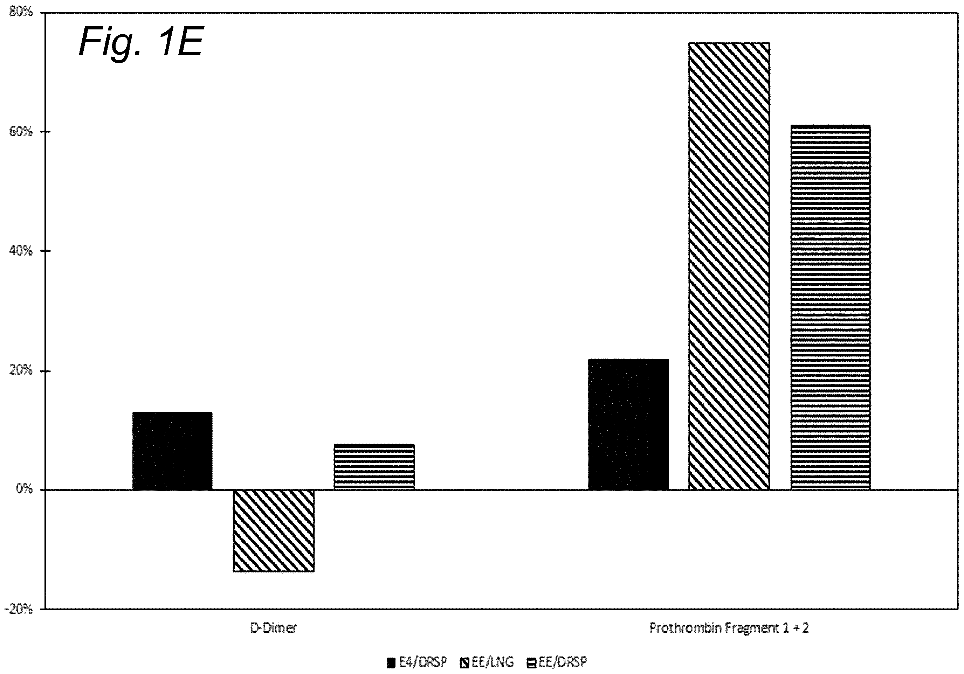

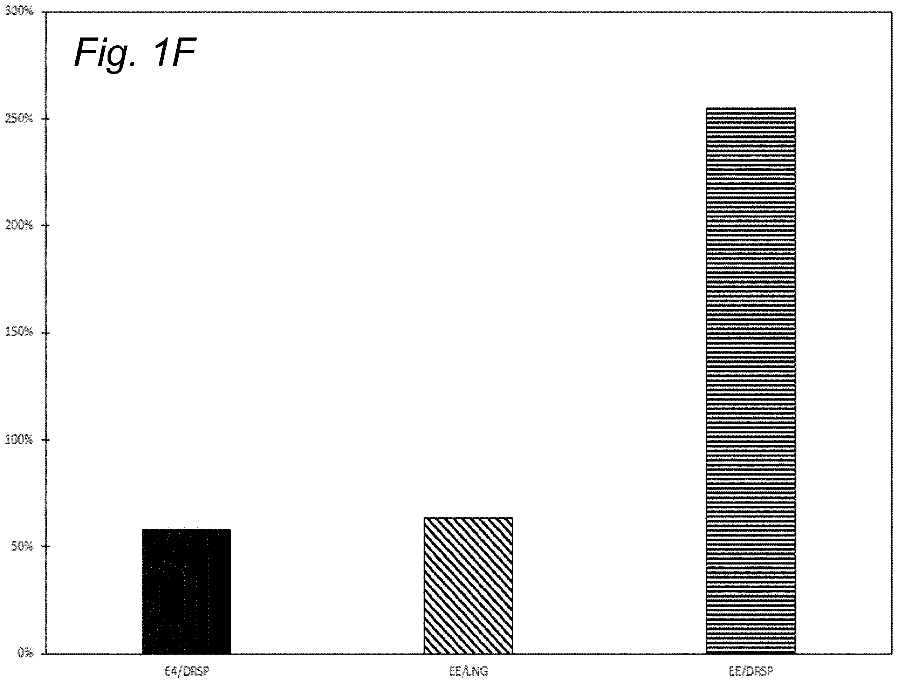

Figures 1A to 1F present the changes observed in 20 haemostasis parameters at cycle 6. For each parameter, the mean relative change from baseline to Cycle 6, in percentage, is displayed for the E4/DRSP group (black bars), for the EE/LNG group (oblique lines) and for the EE/DRSP group (horizontal lines). The data also appears in Table 3 in the Example below. Figure 1A presents five parameters which are coagulation factors. Figure 1B presents five parameters which are anticoagulant proteins. Figure 1C presents four parameters which relate to functional clotting tests. Figure 1D presents three parameters which are fibrinolysis proteins. Figure 1E presents two parameters which are markers of ongoing coagulation. And Figure 1F presents the levels observed for SHBG.

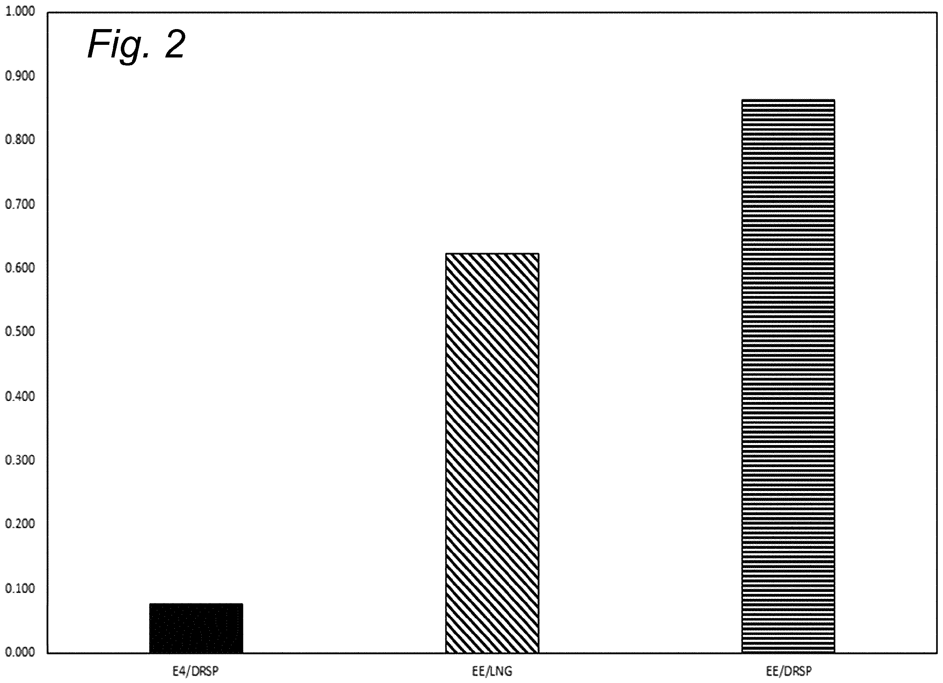

Figure 2 displays the APC-r change (ETP) between Cycle 6 and baseline. The data also appears in Table 1 in the Example below.

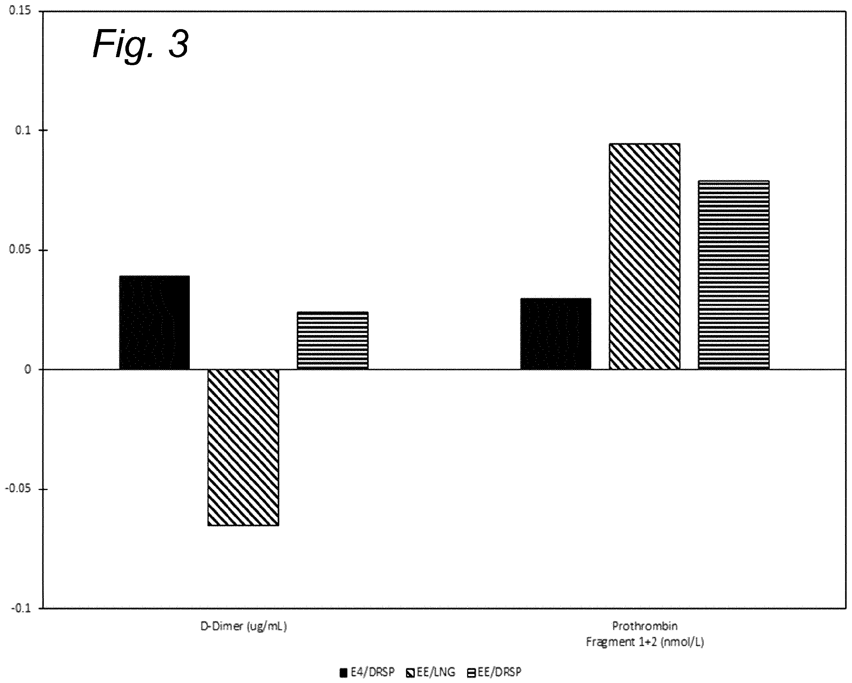

Figure 3 displays the change of D-Dimer and prothrombin fragment 1+2 between Cycle 6 and baseline. The data also appears in Table 1 in the Example below.

### Detailed description of the invention

### Definitions

The term "estetrol component", as used throughout this document, encompasses substances selected from the group consisting of estetrol, esters of estetrol wherein the hydrogen atom of at least one of the hydroxyl groups has been substituted by an acyl radical of a hydrocarbon carboxylic, sulfonic acid or sulfamic acid of 1-25 carbon atoms; and combinations thereof. Even more preferably, the estetrol component is estetrol (including estetrol hydrates). Most preferably, the estetrol component contained in the dosage unit is estetrol monohydrate.

The term "progestogenic component" is defined as a substance that is capable of triggering a progestogenic response in vivo or a precursor which is capable of liberating such a substance in vivo. Usually progestogenic components are capable of binding to a progestogen receptor.

"About" as used herein referring to a measurable value such as a parameter, an amount, a temporal duration, and the like, is meant to encompass variations of +/-10% or less, more preferably +/-5% or less, even more preferably +/-1% or less of and from the specified value, in so far such variations are appropriate to perform in the disclosed invention. However, it is to be understood that the value to which the modifier "about" refers is itself also specifically disclosed.

The term "an effective amount" refers to an amount necessary to obtain a physiological effect. The physiological effect may be achieved by one dose or by repeated doses.

As used herein, the terms 'blood clot risk", are taken as equivalent to "thromboembolism risk" and to "venous thromboembolism risk".

The terms "2^{nd} generation COC", as used herein, refers to a COC combining ethinylestradiol as the estrogen with levonorgestrel (LNG) or norgestrel as the progestogenic component.

The terms "3^{rd} generation COC", as used herein, refers to a COC using gestodene, desogestrel or norgestimate as the progestogenic component.

The terms "4^{th} generation COC", as used herein, refers to a COC using cyproterone acetate, drospirenone, dienogest, nomegestrol or chlormadinone as the progestogenic component.

As used herein, "positive family history", when used in the context of risk factors, refers to family history of venous or arterial thromboembolism or pulmonary embolism, known thrombogenic mutations (by way of example, but not limitation, Factor V Leiden; prothrombin mutation; protein S, protein C and antithrombin deficiencies) which are considered as risk factors for venous thromboembolism in combined oral contraceptive users.

As used herein, "positive personal history", when used in the context of risk factors, refers to personal history of venous or arterial thromboembolism or pulmonary embolism, which are considered as a risk factor for venous thromboembolism in combined oral contraceptive users.

As used herein, "older age", when used in the context of risk factors, refers to an age over 35 years.

As used herein a woman is a "first-ever user", when she is in her first year of ever using CHC. This is generally understood to be a period of increased VTE risk.

As used herein a woman is a "re-starter" with a break of >4 weeks when she has discontinued use of CHC for 4 weeks or more. There is some evidence that the VTE risk is increased when CHC is re-started after a break in use of 4 weeks or more.

As used herein a woman is a "switcher" when she discontinues the use of one type of CHC to initiate the use of another type of CHC. Optionally, this switch comprises a break in use of 4 weeks or more.

As used herein, "BMI" stands for Body Mass Index, and is defined as the body mass divided by the square of the body height, and is expressed in units of kg/m², resulting from mass in kilograms and height in metres.

As used herein, "COC" stands for Combined Oral Contraceptive and "CHC" stands for Combined Hormonal Contraceptive. Throughout this patent application these two terms are used interchangeably, such that embodiments describing COC(s) should be read as embodiments describing CHC(s) and vice-versa. In particular, 2^{nd}, 3^{rd} and 4^{th} generation COCs are equivalent to 2^{nd}, 3^{rd} and 4^{th} generation CHCs, respectively.

As illustrated in the Example, the present contraceptive method has proved to have a surprisingly safe profile, especially by direct comparison to contraceptive methods employing either 4^{th} generation or 2^{nd} generation COCs.

Another important benefit of the present estetrol component is derived from its relative insensitivity to interactions with other drugs (drug-drug interactions). It is well known that certain drugs may decrease the effectiveness of estrogens, such as ethinyl estradiol, and other drugs may enhance their activity, resulting in possible increased side-effects. Similarly estrogens may interfere with the metabolism of other drugs. In general, the effect of other drugs on estrogens is due to interference with the absorption, metabolism or excretion of these estrogens, whereas the effect of estrogens on other drugs is due to competition for metabolic pathways.

The clinically most significant group of estrogen-drug interactions occurs with drugs that may induce hepatic microsomal enzymes which may decrease estrogen plasma levels below therapeutic level (for example, anticonvulsant agents; phenytoin, primidone, barbiturates, carbamazepine, ethosuximide, and methosuximide; antituberculous drugs such as rifampin; antifungal drugs such as griseofulvin). The present estrogenic substances are not dependent on up- and downregulation of microsomal liver enzymes (e.g. P450's) and also are not sensitive to competition with other P450 substrates. Similarly, they do not interfere significantly in the metabolism of other drugs.

In particular, estetrol at a high concentration of 10 µmol/l does not inhibit (less than 10%) the major cytochrome P450 enzymes (CYP1A2, CYP2C9, CYP2C19, CYP2D6 and CYP3A4) unlike estradiol. Indeed, estradiol exerts a substantial inhibitory effect on CYP2C19 and CYP1A2 of 63 % and 19%, respectively. Similarly, ethinyl estradiol, which is the estrogen used in a large number of COCs, exerts a substantial inhibitory effect on CYP2C19 and CYP3A4 of 82 % and 45%, respectively.

The above observations serve to explain why the estetrol component of the invention hardly suffer from drug-drug interactions and thus produce a very consistent, i.e. predictable, impact. Thus, the efficacy of the estetrol substances of the invention is highly reliable.

Additionally, the terminal half-life of the naturally occurring estrogens ranges from 2 to 14 hours while estetrol is characterized by a terminal half-life of 31.7 hours. Consequently, the use of estetrol in the method of the invention allows for a more than 24-hour coverage of the receptors by the treatment. This pharmacokinetic property enhances the efficacy of the product even in case of low treatment compliance by the user.

It has to be noted that when estetrol (E4) is associated with 3 mg drospirenone (DRSP), the bleeding profile and the cycle control is improved in comparison to other combined oral contraceptives using a physiological estrogen, namely estradiol-valerate (E2V) or estradiol (E2).

In a study evaluating the bleeding pattern and cycle control of different E4/DRSP combinations in comparison to a marketed quadriphasic combined oral contraceptive containing E2V and desogestrel (DSG), the combination of 15 mg E4/DRSP was associated with a lower incidence of unscheduled bleeding/spotting days than the comparator. In addition, absence of withdrawal bleeding (also called amenorrhea) was much lower with the E4 containing preparations, particularly E4 associated with DRSP, than with the comparator. Finally, mean number of days with unscheduled bleeding/spotting by cycle was also lower with the combination of 15 mg E4/DRSP in comparison with the E2V/DNG preparation. This was also the case when compared to publicly available data on a marketed combined oral contraceptive containing E2 as estrogen in association with nomegestrol acetate (NOMAC).

Besides, the daily use of currently marketed estrogens (ethinylestradiol (EE), E2, E2V, conjugated equine estrogens) is associated with a dose-proportional increase in triglycerides levels. In the human body, high levels of triglycerides in the bloodstream have been linked to atherosclerosis and, by extension, the risk of heart disease and stroke. In the opposite to the currently available estrogens, E4 minimally increases triglycerides levels even at higher dosages.

As illustrated in the Example, the changes in the surrogate markers of VTE were minimal in comparison to the changes observed with Yaz^{®} (a combination of 20 µg EE and 3 mg DRSP). DRSP is a fourth generation progestin associated with the highest risk of VTE when it is combined with the synthetic estrogen EE. Accordingly, the changes in the surrogate markers of VTE seen with a combination of EE and DRSP are substantial. In comparison, the changes observed with the estetrol component combination are minimal even though DRSP is associated to the estetrol component.

More importantly, it can be seen in the Example that the estetrol and DRSP combination of the invention compares favourably to the 2^{nd} generation COC comparator, consisting of 30 mcg of EE with 150 mcg of LNG (Melleva^{®}). In view of the previously recognized doubling in VTE risk when LNG is substituted by DRSP, it is highly surprising to find such a result.

In particular, parameters such as APC resistance (for example ETP-based APC resistance) is mostly one order of magnitude lower (mean percentage change in Tables 2 and 3 below and in Table 5) with the estetrol-based regimen by comparison with ethinyl estradiol-based regimens.

Acquired activated protein C resistance (by opposition with mutation-related) has been shown to be an independent risk factor of venous thrombosis which increases in the population of COC users (Rosing et al., 1997, Br. J. Haematol. 97, 233-8). APC resistance determined with an ETP-based assay (like in the present application) is an excellent marker for the thrombogenicity of COC.

In addition, as can be seen in Tables 1 and 4 below, statistical significance (for the change from Baseline to Cycle 6) was found between the COC of the invention and both 2^{nd} generation (Melleva^{®}) and 4^{th} generation (Yaz^{®}) COCs for the following parameters: Plasminogen (%), t-PA (ng/mL), ETP-based APC-r, Prothrombin Fragment 1+2 (nmol/L), and Soluble E-Selectin (ng/mL).

Statistical significance (for the change from Baseline to Cycle 6) was found between the COC of the invention and 4^{th} generation (Yaz^{®}) COC for the following parameters: Factor VII activity (%), Protein S (%), Protein S. free (%), Protein C activity (%), aPTT-APC (sec) and SHBG (nmol/L).

### Methods of Treatment

The present methods usually employ uninterrupted oral administration of the estetrol component and the progestogenic component during a period of at least 10 days, preferably of at least 20 days.

The term "uninterrupted" as used in here, means that the components are administered at relatively regular intervals, with no (therapeutically) significant interruptions. Naturally, minor interruptions may occur that do not affect the overall effectiveness of the present method, and indeed such aberrations are encompassed by the present invention. In a preferred embodiment, and more arithmetically, the administration regimen is deemed to be continuous if the longest interval between 2 subsequent administrations is not more than 3.5 times as long as the average interval. Even more preferably said longest interval is not more than 2.5 times, most preferably not more than 1.5 times as long as the average interval.

In the present method, the estetrol and progestogenic components may be administered in separate dosage units. However, it is also possible and indeed very convenient to combine these two components into a single dosage unit.

In the method according to the present invention the combination of the progestogenic and estetrol component is suitably administered uninterruptedly during a period of at least 10 days.

The invention may suitably be reduced to practice in the form of a variety of administration methods that are known to the person skilled in the art. Amongst these methods are the so called "combined" methods. The combined methods make use of monophasic preparations, which contain dosage units with a constant amount of an estrogen and a progestogen, or bi- or triphasic preparations which have varying levels of estrogen and progestogen; in most cases consisting of relatively constant levels of estrogen with a step-wise increase in progestogen throughout the cycle. The combined methods have in common that they are based on a regimen which involves an administration-free interval of about 7 days whereby withdrawal bleeding, simulating the natural menses, occurs. Thus 21 day intervals of hormone administration alternate with 7 days during which no hormones are administered.

In a preferred embodiment of the method of the invention, an administration-free interval of about 4 days is used. In this embodiment, a 24 day interval of hormone administration alternates with 4 days during which no hormones are administered.

In yet another preferred embodiment of the method of the invention, a 24 day interval of hormone administration during which an estetrol component and a progestogenic component are administered alternates with 4 days during which only an estetrol component is administered (from day 25 to day 28).

As an alternative to the aforementioned combined methods, the so called "sequential" method has been proposed. Typical of the sequential method is that it comprises two consecutive phases, i.e. one phase during which estrogen and no progestogen is administered and another phase during which a combination of estrogen and progestogen is administered. The first sequential methods, like the aforementioned combined methods, made use of an administration free interval of about 7 days. More recently, sequential methods have been proposed which do not include an administration-free (or placebo) period, meaning that estrogen is administered throughout the full cycle and that progestogen is co-administered during only part of that cycle. WO 95/17895 (Ehrlich et al.) describes such an uninterrupted sequential method.

Yet another example of a method which is encompassed by the present invention is the so called "continuous combined" method, which is a particular version of the combined method that uses uninterrupted combined administration of a progestogenic and an estrogenic component during a prolonged period of time, e.g. more than 50 days. In contrast to ordinary combined and sequential methods, no regular menses occur in the continuous combined method as the continuous administration of progestogen in the indicated amounts induces amenorrhoea.

In one embodiment of the invention, which relates to the continuous combined method, the present method comprises the uninterrupted oral administration of the combination of the estetrol component and the progestogenic component during a period of at least 28, preferably at least 60 days.

In one specific embodiment of the continuous combined method according to the invention, one tablet comprising the combination of the estetrol component and of the progestogenic component is initially taken daily for at least 24 consecutive days. Subsequently, during days 25 to 120, the patient may decide to take a 4-day tablet-free break. Said tablet-free break may not be longer than 4 days. In any case, a 4-day tablet-free break has to be taken after 120 days of continuous tablet administration. After each 4-day tablet-free break, a new cycle starts with a minimum of 24 days and a maximum of 120 days of continuous administration.

In another embodiment of the invention, which relates to sequential and combined methods that employ a significant administration-free interval, the method of the invention comprises an interval of at least 2 days, preferably from 3-9 days, most preferably from 5-8 days, during which no progestogenic component and no estetrol component is administered and wherein the resulting decrease in serum concentration of the progestogenic component and the estetrol component induces menses.

Yet another embodiment of the invention, which concerns a sequential method without a significant pause, is characterised in that it comprises the uninterrupted oral administration of the estetrol component during a period of at least 28 days, preferably at least 60 days, and in that, following the combined administration of the estetrol component and the progestogenic component, the estetrol component and no progestogenic component are administered during 3-18 consecutive days, preferably during 5-16 consecutive days and the resulting decrease in serum concentration of the progestogenic component should normally be sufficient to induce menses.

According to the present invention, the contraceptive composition is capable of reducing the number, frequency and/or severity of adverse side effects including VTE, ATE.

In a particular embodiment of the invention, the method does not cause haemostatic change that exceeds the boundaries of the normal range. As used herein, "haemostatic change" is defined as the variation, upon administration of the compositions according to the invention, of the plasma level of one or more markers selected from: Sex Hormone Binding Globulin (SHBG), free tissue factor pathway inhibitor (free TFPI), free and total protein-S, protein-S activity, Corticosteroid Binding Globulin (CBG), Ceruloplasmin, antithrombin III, activated protein C (APC) resistance (e.g. APTT-based APCr or ETP-based APCr), Protein-C activity, D-dimer, Prothrombin, Prothrombin activity, Prothrombin fragment 1+2, Factor VII, Factor VIII, von Willebrand factor, Factor II, PAI-1, tissue-type plasminogen (t-PA), plasminogen, E-selectin, and fibrinogen.

The above-listed markers are well-known to the skilled person and methods for the determination of their level are within the common general knowledge of the skilled person.

As used herein, the "normal range", when referring to levels of haemostatic markers, refers to the prediction interval that 95% of the population fall into.

In one embodiment of the invention, the method does not cause haemostatic change exceeding the boundaries of the normal range after one cycle of treatment, preferably the method does not cause haemostatic change exceeding the boundaries of the normal range after two cycles of treatment, even more preferably the method does not cause haemostatic change exceeding the boundaries of the normal range after three cycles of treatment.

In another particular embodiment of the invention, the method does not cause a change in the level of protein-S which exceeds the boundaries of the normal range.

In another particular embodiment of the invention, the method does not cause a change in the level of free TFPI which exceeds the boundaries of the normal range.

In a preferred embodiment, the contraceptive method of the invention which provides a better safety profile than currently available COCs, and in particular which displays a lower risk of thromboembolic events, is employed in at-risk populations, such as first-ever users; switchers/re-starters with a break of >4 weeks; and women with increased baseline risk for VTE due to one or more major risk factors (such as, but not limited to, BMI>30, older age, positive personal and/or family history, and risk factors such as those identified in Anderson and Spencer, 2003, Circulation, 107:1-9 -I-16, *"Risk factors for venous thromboembolism"* which include, by way of example and not limitation, major surgery, prolonged immobility, childbirth, postpartum).

In relation to the at-risk population of older age, it is important to note that, as reported in Lidegaard et al. (2011) BMJ 343:d6423 (*"Risk of venous thromboembolism from use of oral contraceptives containing different progestogens and oestrogen doses: Danish cohort study, 2001-9"),* in particular in their Table 1 on page 9, VTE risk in non-users increases from 0.7 per 10,000 women year in the 15-19 years population to 5.8 per 10,000 women year in the 45-49 years population. For this latter population, COC use increases VTE risk by a factor of 3.6 (Table 1 of Lidegaard et al. 2011). The advantageous use of a COC according to the invention is beneficial across all age groups, but significantly more so for the older age groups (e.g., age over 35) which have the highest incidence in absence of COC use, and whose incidence is additionally the most affected by use of a COC of the prior art.

### Compositions

The estetrol component of the present invention encompasses substances selected from the group consisting of estetrol, esters of estetrol wherein the hydrogen atom of at least one of the hydroxyl groups has been substituted by an acyl radical of a hydrocarbon carboxylic, sulfonic acid or sulfamic acid of 1-25 carbon atoms; and combinations thereof. More preferably, the estetrol component is estetrol (including estetrol hydrates). Most preferably, the estetrol component contained in the dosage unit is estetrol monohydrate.

The estetrol component of the invention may be used at a daily dose of from 0.1 mg to 100 mg. Preferably, the estetrol component of the invention is used at a daily dose of from 1 mg to 40 mg. Even more preferably, the estetrol component of the invention is used at a daily dose of from 5 mg to 25 mg. Still more preferably, the estetrol component of the invention is used at a daily dose of from 10 mg to 20 mg.

In a most preferred embodiment, the estetrol component of the invention is used at a daily dose of about 15 mg.

In other embodiments, dosages may be variable throughout the cycle (bi-phasic, triphasic or quadriphasic administration).

In a particularly preferred embodiment of the invention the pharmaceutical composition according to invention is designed for daily administration, i.e. it represents a daily dosage unit.

In the case of oral administration, the oral dosage unit according to the invention is preferably a solid or semi-solid dosage form such as tablets, capsules, cachets, pellets, pills, powders and granules. The term "solid or semi-solid dosage form" also encompasses capsules that contain a liquid, e.g. an oil, in which the present estetrol component is dissolved or dispersed. Tablets and equivalent solid and semi-solid dosage forms can suitably contain materials such as binders (e.g. hydroxypropylmethyl cellulose, polyvinyl pyrrolidone, other cellulosic materials and starch), diluents (e.g. lactose and other sugars, starch, dicalcium phosphate and cellulosic materials), disintegrating agents (e.g. starch polymers and cellulosic materials) and lubricating agents (e.g., stearates and talc). These tablets and equivalent solid and semi-solid dosage forms may be prepared by wet granulation, e.g. using an aqueous solution or an organic solution, as well as by direct compression.

Examples of progestogenic components which may suitably be used in accordance with the present invention include: levonorgestrel, norgestimate, norethisterone, dydrogesterone, drospirenone, 3-beta-hydroxydesogestrel, 3-ketodesogestrel, 17-deacetylnorgestimate, 19-norprogesterone, acetoxypregnenolone, allylestrenol, amgestone, chlormadinone, cyproterone, demegestone, desogestrel, dienogest, dihydrogesterone, dimethisterone, ethisterone, ethynodiol diacetate, fluorogestone acetate, gastrinone, gestodene, gestrinone, hydroxymethylprogesterone, hydroxyprogesterone, lynestrenol, mecirogestone, medroxyprogesterone, megestrol, mele,gestrol, nomegestrol, norethindrone, norethynodrel, norgestrel (including d-norgestrel, and dl-norgestrel), norgestrienone, normethisterone, progesterone, quingestanol, (17 alpha)-17-hydroxy-11-methylene-19-norpregna-4, 15-dien-20-yn-3-one, tibolone, trimegestone, algestone-acetophenide, nestorone, promegestone, 17-hydroxyprogesterone esters, 19-nor-17hydroxyprogesterone, 17alpha-ethynyltestosterone, 17alpha-ethynil-19-nortestosterone, d-17beta-acetoxy-13beta-ethyl-17alpha-ethynylgon-4-en-3-one oxime, 6beta, 7beta;15beta,16beta-dimethylene-3-oxo-17-pregna-4,9(11)-diene-21, 17beta-carbolactone or tanaproget and precursors of these compounds that are capable of liberating these progestogens in vivo when used in the present method.

Preferably the progestogenic component used in the present method is selected from the group consisting of progesterone, desogestrel, gestodene, dienogest, levonorgestrel, norgestimate, norethisterone, drospirenone, trimegestone, dydrogesterone, precursors of these progestogens and mixtures thereof.

When the progestogenic component of the invention is drospirenone, it is preferably used at a daily dose of from 0.5 mg to 10 mg, even more preferably of from 1 mg to 4 mg. In a most preferred embodiment, the progestogenic component of the invention is drospirenone and it is used at a daily dose of about 3 mg.

When a different progestogenic component is used, the daily dose is adjusted such as to give the same pharmacological effect as a dose of 0.5 mg to 10 mg of drospirenone, preferably to give the same pharmacological effect as a dose of 1 mg to 4 mg of drospirenone.

In a preferred embodiment of the invention, the composition combines estetrol at a daily dose of from 5 mg to 25 mg with drospirenone at a daily dose of 0.5 mg to 10 mg. In a more preferred embodiment of the invention, the composition combines estetrol at a daily dose of from 10 mg to 20 mg with drospirenone at a daily dose of 1 mg to 4 mg. In a yet more preferred embodiment of the invention, the composition combines estetrol at a daily dose of about 15 mg with drospirenone at a daily dose of about 3 mg. In another embodiment, the composition combines estetrol at a daily dose of about 5 mg with drospirenone at a daily dose of about 3 mg. In yet another embodiment, the composition combines estetrol at a daily dose of about 10 mg with drospirenone at a daily dose of about 3 mg.

The present invention has been described above with reference to a number of exemplary embodiments. Modifications and alternative implementations of some parts or elements are possible, and are included in the scope of protection as defined in the appended claims.

### Example

### Study Protocol

A single center, randomized, open-label, controlled, three-arm study to evaluate the effect of a Combined Oral Contraceptive (COC) containing 15 mg estetrol (E4) and 3 mg drospirenone (DRSP) and of two reference COCs containing either 30 mcg ethinylestradiol (EE) and 150 mcg levonorgestrel (LNG) or 20 mcg EE and 3 mg DRSP on endocrine function, metabolic control and haemostasis during 6 treatment cycles was conducted.

| | |
|---|---|
| Study Type : | Interventional (Clinical Trial) |
| Actual Enrollment: | 101 participants |
| Allocation: | Randomized |
| Intervention Model: | Parallel Assignment |
| Masking: | None (Open Label) |
| Primary Purpose: | Prevention |

| **Arm** | **Intervention/treatment** |
|---|---|
| Experimental: 15 mg E4/3 mg DRSP combined oral contraceptive | 15 mg E4 combined with 3 mg DRSP administered in a 24/4-day regimen (i.e. 24 days of pink active tablets followed by 4 days of white placebo tablets). One tablet per day orally for 6 treatment cycles. |
| Active Comparator: 30 mcg EE/150 mcg LNG combined oral contraceptive | 30 mcg EE combined with 150 mcg LNG administered in a 21/7-day regimen (i.e. 21 days of yellow active tablets followed by 7 days of white placebo tablets). One tablet per day orally for 6 treatment cycles. |
| Active Comparator: 20 mcg EE/3 mg DRSP combined oral contraceptive | 20 mcg EE combined with 3 mg DRSP administered in a 24/4-day regimen (i.e. 24 days of pink active tablets followed by 4 days of white placebo tablets). One tablet per day orally for 6 treatment cycles. |

### Primary Outcome Measures :

For each of the parameters 1 to 16 listed below, the following applies:
[ Time Frame: Between Days 18 and 21 for the pretreatment Cycle, and between Days 18 and 21 for the Cycles 3 and 6 (1 cycle = 28 days). ]
1. Plasma concentration of prothrombin fragment 1+2
2. Plasma concentration of APC resistance (ETP-based, APTT-based)
3. Plasma concentration of D-dimer
4. Plasma concentration of factor VII
5. Plasma concentration of factor VIII
6. Plasma concentration of von Willebrand factor
7. Plasma concentration of factor II
8. Plasma concentration of antithrombin
9. Plasma concentration of free and total Protein S
10. Plasma concentration of protein C
11. Plasma concentration of plasminogen activator inhibitor type-1 (PAI-1)
12. Plasma concentration of tissue type plasminogen activator (t-PA)
13. Plasma concentration of plasminogen
14. Plasma concentration of free tissue factor pathway inhibitor (TPFI)
15. Plasma concentration of E-selectin
16. Plasma concentration of fibrinogen

For each of the parameters 17 to 21 listed below, the following applies:
[ Time Frame: At screening, between Days 18 and 21 for the pretreatment Cycle, and between Days 18 and 21 for Cycles 3 and 6 (1 cycle = 28 days). ]
17. Serum concentration of insulin
18. Serum concentration of glucose
19. Serum concentration of C-peptide
20. Plasma concentration of glycated hemoglobin (HbA1c)
21. Homeostasis Model Assessment - Insulin Resistance (HOMA-IR)
22. Oral glucose tolerance test (OGTT) [ Time Frame: At 0 (pre-glucose challenge), 30, 60, 90, 120 and 180 minutes after glucose challenge during pretreatment Cycle; at 0 (pre-glucose challenge), 30, 60, 90, 120 and 180 minutes after glucose challenge during Cycles 3 and 6 (1 cycle = 28 days). ]

For each of the parameters 23 to 35 listed below, the following applies:
[ Time Frame: Between Days 18 and 21 for the pretreatment Cycle, and between Days 18 and 21 for Cycles 3 and 6 (1 cycle = 28 days). ]
23. Serum concentration of prolactin
24. Serum concentration of follicle-stimulating hormone (FSH)
25. Serum concentration of luteinizing hormone (LH)
26. Serum concentration of estradiol (E2)
27. Serum concentration of progesterone (P)
28. Serum concentration of thyroid stimulating hormone (TSH)
29. Serum concentration of free thyroxine (fT3)/free triiodothyronine (fT4)
30. Serum concentration of dihydroepiandrostenedione (DHEAS)
31. Serum concentration of androstenedione
32. Serum concentration of total testosterone (T)
33. Serum concentration of dihydrotestosterone (DHT)
34. Serum concentration of total cortisol
35. Serum concentration of aldosterone

For each of the parameters 36 to 39 listed below, the following applies:
[ Time Frame: At screening, between Days 18 and 21 for the pretreatment Cycle, and between Days 18 and 21 for the Cycles 3 and 6 (1 cycle = 28 days). ]
36. Serum concentration of high density lipoprotein (HDL)-cholesterol
37. Serum concentration of low density lipoprotein (LDL)-cholesterol
38. Serum concentration of total cholesterol
39. Serum concentration of triglycerides

For each of the parameters 40 to 47 listed below, the following applies:
[ Time Frame: Between Days 18 and 21 for the pretreatment Cycle, and between Days 18 and 21 for the Cycles 3 and 6 (1 cycle = 28 days). ]
40. Serum concentration of lipoprotein (a)
41. Serum concentration of apoliporotein A1
42. Serum concentration of apoliporotein B
43. Serum concentration of C-reactive protein
44. Serum concentration of corticosteroid binding globulin (CBG)
45. Serum concentration of sex hormone binding globulin (SHBG)
46. Serum concentration of thyroxin binding globulin (TBG)
47. Serum concentration of angiotensinogen

### Secondary Outcome Measures :

1. Number of subjects with adverse events as a measure of safety and tolerability
   [ Time Frame: From up to 28 days before randomization to maximum Day 4 of the Cycle 7 (1 cycle = 28 days). ]
2. Serum concentration of lactate dehydrogenase (LDH) 1 and 2
   [ Time Frame: Between Days 18 and 21 for the pretreatment Cycle and between Days 18 and 21 for the Cycle 6 (1 cycle = 28 days) ]
3. Serum concentration of tropinin T and I [ Time Frame: Between Days 18 and 21 for the pretreatment Cycle and between Days 18 and 21 for the Cycle 6 (1 cycle = 28 days) ]
4. Electrocardiogram (ECG) parameters [ Time Frame: At screening and between Days 18 and 21 for Cycle 6 (1 cycle = 28 days). ]
   The following ECG parameters will be recorded: heart rate, PR-interval, QRS-duration, QT-interval, QTc interval (Fridericias's)
5. Echocardiographic parameters [ Time Frame: At screening and between Days 18 and 21 for Cycle 6 (1 cycle = 28 days). ]
6. Change from baseline to end of treatment in the different items of the menstrual distress questionnaires (MDQ) form C [ Time Frame: At pretreatment Cycle and between Days 18 and 21 for Cycle 6 (1 cycle = 28 days). ]

### Eligibility Criteria

| | |
|---|---|
| Ages Eligible for Study: | 18 Years to 50 Years (Adult) |
| Sexes Eligible for Study: | Female |
| Accepts Healthy Volunteers: | Yes |

### Criteria

### Inclusion Criteria:

- Healthy adult woman
- Negative pregnancy test at subject screening and randomization
- Aged 18-50 years (inclusive) at the time of signing the ICF
- Good physical and mental health on the basis of medical, surgical and gynecological history, physical examination, gynecological examination, clinical laboratory, ECG, echocardiography and vital signs
- BMI from 18.0 to 30.0 kg/m², inclusive, at time of screening visit
- Able to fulfil the requirements of the protocol and have indicated a willingness to participate in the study by providing written informed consent

### Exclusion Criteria:

- Known hypersensitivity to any of the investigational product ingredients
- Smoking if > 35 years old
- Dyslipoproteinemia or use of antilipidemic agent
- Known diabetes mellitus
- Current use of antidiabetic drugs, including insulin
- Arterial hypertension
- Any condition associated with an increased risk of venous thromboembolism and/or arterial thromboembolism.
- Any condition associated with abnormal uterine/vaginal bleeding.
- Presence of an undiagnosed breast mass
- Current symptomatic gallbladder disease
- History of pregnancy- or COC-related cholestasis
- Presence or history of severe hepatic disease
- Presence or history of pancreatitis if associated with hypertriglyceridemia
- Porphyria
- Presence or history of benign liver tumors (focal nodular hyperplasia and hepatocellular adenoma)
- Presence of renal impairment (glomerular filtration rate [GFR] <60 mL/min/1.73m²)
- Hyperkalemia or presence of conditions that predispose to hyperkalemia
- Presence or history of hormone-related malignancy
- History of non-hormone-related malignancy within 5 years before screening; subjects with a non-melanoma skin cancer are allowed in the study
- Use of drugs potentially triggering interactions with COCs
- History of alcohol or drug abuse within 12 months prior to screening
- Presence or history of thyroid disorders
- Participation in another investigational drug clinical study within 1 month (30 days) or have received an investigational drug within the last 3 months (90 days) prior to randomization. Subjects who participated in an oral contraceptive clinical study using Food and Drug Administration (FDA)/European Union (EU) approved active ingredients, may be randomized 2 months (60 days) after completing the preceding study
- Sponsor, contract research organization (CRO) or Principal Investigator's (PI's) site personnel directly affiliated with this study
- Is judged by the PI to be unsuitable for any reason

### Results

The following haemostasis parameters were determined at base line (no COC use) and after 3 cycles and 6 cycles of COC use:
Coagulation factors: fibrinogen, prothrombin, factor VII, factor VIII and von Willebrand factor (note: although classified in this group for ease of reference, the van Willebrand factor is actually not, *per se,* a coagulation factor).
Anticoagulant proteins: antithrombin, protein S (ELISA and activity), factor XIV = protein C, and TFPI.
Proteins involved in fibrinolysis: plasminogen, tissue plasminogen activator (tPA), plasminogen activator inhibitor-1 (PAI-1)
Functional clotting tests: aPTT-based activated protein C resistance (APCr), ETP-based activated protein C resistance, activated coagulation time - APC, activated coagulation time + APC.
Markers of ongoing coagulation: prothrombin fragment 1+2 and D-Dimer Miscellaneous: soluble E-selectin and sex hormone binding globulin (SHBG).

With respect to the thrombogenicity of the COCs, the haemostasis parameters that are associated with an increased risk of venous thrombosis or that are so-called (surrogate) risk markers of venous thrombosis are:
Coagulation factors: fibrinogen, prothrombin, and factor VIII
Anticoagulant proteins: antithrombin, protein S (ELISA and activity), factor XIV = protein C, and TFPI.
Proteins involved in fibrinolysis: are in general not considered to be risk factors of VTE.
Functional clotting tests: aPTT-based activated protein C resistance (APCr), ETP-based activated protein C resistance
Markers of ongoing coagulation: prothrombin fragment 1+2 and D-Dimer
Miscellaneous: sex hormone binding globulin (SHBG) is a surrogate marker of VTE risk in case of hormone use.

In the context of prothrombin, of particular interest is the publication by Poort et al. (A common genetic variation in the 3'-untranslated region of the prothrombin gene is associated with elevated plasma prothrombin levels and an increase in venous thrombosis, Blood, Vol. 88, N°10, 1996, pages 3698-3703). Table 2 on page 3701 of this publication establishes a relationship between the levels of prothrombin activity and the Odds Ratio for thrombosis risk. From this table, it can for example be seen that in case the prothrombin activity is increased by a level of between 5% to 15% above the normal (which is taken as 100%), the odds ratio of thrombosis risk is at 1.4. When the prothrombin activity level rises above 115%, the odds ratio changes to 2.1.

Because the actual population is in fact spread around the average value in a Gaussian distribution, apparently small changes in the position of the centre of the Gaussian (the average prothrombin level) imply significant changes in the number of subjects in the high risk category (the category appearing last in the Table 2 from Poort et al., i.e. the subjects displaying 115% and more as prothrombin level and thus having a 2.1 Odds ratio). For example, while in a group of subjects not taking COCs (and therefore displaying an average prothrombin level at 100%), the "tail" of the Gaussian curve corresponding to subjects at high risk (with an Odds Ratio of 2.1) represents 14% of the whole population, in a group of subjects taking a COC which displaces the average prothrombin level to 111% (as is the case in the data of the Example for the EE/LNG treatment, please refer to the "prothrombin activity" entry in Table 3 at cycle 6), the high risk patient population (with an Odds Ratio of 2.1) will very significantly increase to 38% of the whole population.

From this illustrative calculation, it can easily be understood that a small variation in the average level of a thrombotic parameter may in fact have a very large impact in terms of the number of subjects falling into the high risk category.

To summarise the effects the different COCs on the haemostasis parameters, for each parameter a number of different approaches were used. In a first method, the changes of the average measured levels amongst the subjects in a given group were calculated at cycle 3 and cycle 6 by comparison to the average levels at the baseline. The corresponding values are displayed in Table 1 below.

In a second approach, the changes at cycles 3 and 6 by comparison to the baseline were initially computed for each patient, and the averages of these changes was then calculated. The corresponding results are presented as percentages in Table 2 below.

Thirdly, the average measured levels at baseline and at cycles 3 and 6 were used to calculate the percentage of changes from baseline to cycles 3 and 6 which are presented in Table 3 below.

In the present study, the effects of EE/DRSP on haemostasis parameters are more pronounced than those of EE/LNG. Very surprisingly, however, the effects of E4/DRSP are comparable to those of EE/LNG (second generation COC). Actually, for most parameters the effect of E4/DRSP is even less than that of EE/LNG which suggests that E4/DRSP might be less thrombogenic than EE/LNG.

In relation to the Odds Ratio from the Poort et al. paper discussed above, it is important to note in Table 3 that the Prothrombin activity is found to increase by only 5.9% after 6 cycles when the method of the invention is used, whereas it is found to increase by 11,2% and 10,4 % when EE/LNG and EE/DRSP are used, respectively. From this point of view, the method of the invention is shown to put a lot fewer subjects into the "at risk" category (defined as having an Odds Ratio of 2.1).

Similarly, it is known from Odlind et al. (Can changes in sex hormone binding globulin predict the risk of venous thromboembolism with combined oral contraceptive pills?, Acta Obstet Gynecol Scand, 2002; 81; pages 482-490) that the incidence of VTE is directly linked to the amount of increase in SHBG levels observed after COCs are administered. Based on the levels observed in Table 3, it can be seen that the method of the invention is estimated to induce a slightly lower risk than the EE/LNG COC, and induces a vastly lower risk than the EE/DRSP COC. In other words, the contraceptive method of the invention would be displayed in the Figure 1 from Odlind et al. in the lower left corner of the graph, close to the "LNG" data point, and very remote from the "DSG/GST" and the "CPA" datapoints.

**Table 1. Change from baseline (hereinafter, "BL") to Cycles 3 and 6 in levels of various haemostasis parameters (per-protocol population)**

| **Table 1 - Haemostasis parameters** | | **E4/DRSP** | | | **EE/LNG** | | | **EE/DRSP** | | |
|---|---|---|---|---|---|---|---|---|---|---|
| **Mean (SD)** | | **Baseline** | **Change BL to Cycle 3** | **Change BL to Cycle 6** | **Baseline** | **Change BL to Cycle 3** | **Change BL to Cycle 6** | **Baseline** | **Change BL to Cycle 3** | **Change BL to Cycle 6** |
| **Median (min-max)** | | | | | | | | | | |
| ***Coagulation factors*** | | | | | | | | | | |
| Fibrinogen (mg/dL) | **mean change (SD)** | 246.1 (42.24) | 11.0 (48.27) | 19.9 (40.62) | 254.9 (77.32) | 27.1 (71.74) | 19.9 (73.45) | 241.3 (47.63) | 44.2 (60.06) | 37.8 (59.86) |
| | **median change (min - max)** | 240.0 (173 - 355) | 9.0 (-77 - 162) | 27.0 (-63 - 93) | 239 (144-472) | 22.0 (-179 - 205) | 12.0 (-167-199) | 228.5 (176-409) | 57.5 (-107 - 159) | 36.5 (-182 - 152) |
| Prothrombin activity (%) | **mean change (SD)** | 85.7 (7.40) | 5.2 (7.06) | 5.1 (7.86) | 89.0 (11.42) | 11.7 (7.15) | 9.5 (8.55) | 90.0 (9.44) | 12.9 (7.97) | 9.4 (9.58) |
| | **median change (min - max)** | 86.0 (67 - 106) | 7.0 (-10 - 18) | 5.0 (-10 - 20) | 87.0 (72 - 112) | 11.0 (-1 - 25) | 10.0 (-8 - 31) | 89.0 (77 - 112) | 14.0 (1-27) | 6.5 (-3 - 38) |
| Factor VII activity (%) | **mean change (SD)** | 97.2 (22.80) | -5.1 (11.32) | -1.3 (15.78) | 93.1 (18.26) | -7.1 (14.89) | -8.0 (14.07) | 95.5 (17.59) | 19.9 (19.27) | 17.7 (15.84) |
| | **median change (min - max)** | 94.0 (61 - 166) | -4.0 (-33 - 22) | -3.0 (-34 - 38) | 93.0 (53 - 127) | -7.0 (-46 - 23) | -4.0 (-43 - 25) | 93.5 (64 - 130) | 18.0 (-12 - 68) | 17.5 (-13 - 56) |
| | **p-value*** | | | | | | 0.3711 | | | 0.0001 |
| Factor VIII activity (%) | **mean change (SD)** | 135.4 (47.62) | 10.2 (28.16) | 0.3 (28.91) | 120.9 (34.88) | 8.7 (29.47) | 0.3 (35.79) | 118.0 (33.49) | 20.7 (25.10) | 9.9 (30.56) |
| | **median change (min - max)** | 114.0 (85 - 255) | 11.0 (-62 - 68) | 6.0 (-69 - 65) | 115.0 (74 - 217) | 9.0 (-77 - 72) | 3.0 (-83 - 92) | 110.0 (72 - 203) | 19.0 (-19 - 56) | 7.0 (-53 - 79) |
| vWF (%) | **mean change (SD)** | 109.9 (38.31) | 4.9 (17.14) | 6.0 (20.66) | 105.1 (46.84) | -1.7 (18.94) | -3.6 (20.15) | 93.8 (24.48) | 10.1 (14.32) | 12.9 (19.10) |
| | **median change (min - max)** | 103.0 (60 - 240) | 4.0 (-50 - 38) | 6.0 (-39 - 55) | 96.0 (56 - 295) | -2.0 (-60 - 30) | -2.0 (-60 - 37) | 88.0 (55 - 154) | 8.0 (-13 - 40) | 10.0 (-29 - 58) |
| | **p-value*** | | | | | | 0.1673 | | | 0.4016 |

| ***Anticoagulant proteins*** | | | | | | | | | | |
|---|---|---|---|---|---|---|---|---|---|---|
| Antithrombin (%) | **mean change (SD)** | 97.4 (6.75) | -0.1 (5.41) | 0.2 (5.09) | 98.1 (11.71) | -2.8 (6.33) | -3.9 (6.43) | 98.9 (6.66) | -2.1 (7.06) | -2.2 (8.43) |
| | **median change (min - max)** | 96.0 (87 - 110) | 0.0 (-11 - 12) | -1.0 (-9 - 11) | 99.0 (64 - 124) | -2.0 (-21 - 12) | -5.0 (-13 - 11) | 96.5 (88 - 114) | -2.5 (-13 - 13) | -3.5 (-17 - 15) |
| Protein S (%) | **mean change (SD)** | 94.5 (13.08) | 0.6 (13.77) | -2.3 (12.16) | 104.1 (16.89) | -1.2 (18.26) | -7.4 (15.62) | 104.9 (17.07) | -25.0 (12.16) | -31.8 (13.42) |
| | **median change (min - max)** | 93.0 (74 - 134) | 1.0 (-29 - 36) | -5.0 (-22 - 26) | 105.0 (82 - 142) | -2.0 (-30 - 50) | -5.0 (-41 - 29) | 104.0 (65 - 149) | -26.0 (-57 - -6) | -30.5 (--67 - -8) |
| | **p-value*** | | | | | | 0.4564 | | | <0.0001 |
| Protein S. free (%) | **mean change (SD)** | 85.2 (11.57) | 6.5 (8.96) | 3.7 (10.23) | 95.6 (13.14) | 2.3 (16.58) | -1.1 (14.77) | 91.0 (14.77) | 20.0 (8.67) | -23.3 (14.22) |
| | **median change (min - max)** | 84.0 (59 - 111) | 7.0 (-8 - 27) | 4.0 (-21 - 25) | 94.0 (76 - 121) | 2.0 (-26 - 50) | -3.0 (-23 - 26) | 88.0 (69 - 134) | -20.0 (-34 - 0) | -20.0 -60 - 1) |
| | **p-value*** | | | | | | 0.5045 | | | <0.0001 |
| Protein C activity (%) | **mean change (SD)** | 95.0 (15.42) | 1.5 (9.81) | 1.6 (10.46) | 96.7 (14.84) | 10.6 (11.47) | 9.4 (13.79) | 97.7 (15.19) | 17.7 (12.18) | 18.5 (13.69) |
| | **median change (min - max)** | 95.0 (73 - 141) | 1.0 (-15 - 32) | 2.0 (-19-26) | 98.0 (71 - 135) | 12 (-11 - 31) | 8.0 (-14-36) | 97.5 (69 - 122) | 19.5 (-11 - 54) | 18.0 (0-66) |

| **Table 1 - Haemostasis parameters** | | **E4/DRSP** | | | **EE/LNG** | | | **EE/DRSP** | | |
|---|---|---|---|---|---|---|---|---|---|---|
| **Mean (SD)** | | **Baseline** | **Change BL to Cycle 3** | **Change BL to Cycle 6** | **Baseline** | **Change BL to Cycle 3** | **Change BL to Cycle 6** | **Baseline** | **Change BL to Cycle 3** | **Change BL to Cycle 6** |
| **Median (min-max)** | | | | | | | | | | |
| | **p-value*** | | | | | | 0.0794 | | | <0.0001 |
| TFPI (U/mL) | **mean change (SD)** | 1.108 (0.30) | 0.032 (0.47) | 0.079 (0.27) | 0.141 (0.292) | 0.005 (0.407) | -0.067 (0.3588) | 0.18 (0.328) | -0.133 (0.49) | -0.212 (0.278) |
| | **median change (min - max)** | 1.060 (0.50 - 1.60) | 0.070 (-0.89 - 0.68) | -0.095 (0.84 - 0.55) | 1.030 (0.69 - 1.60) | 0.020 ( -0.57 - 0.80) | -0.090 (-0.90- 0.62) | 1.025 (0.69 - 1.60) | -0.190 (-0.92 - 0.79) | -0.205 (-0.67 - 0.24) |
| ***Fibrinolysis proteins*** | | | | | | | | | | |
| Plasminogen (%) | **mean change (SD)** | 93.5 (10.70) | 10.2 (8.96) | 12.2 (9.15) | 97.8 (13.17) | 39.3 (10.81) | 39.1 (13.20) | 98.2 (11.69) | 35.2 (14.86) | 35.5 (12.78) |
| | **median change (min - max)** | 94.0 (68 - 123) | 12.0 (-12 - 29) | 12.0 (-5 - 33) | 96.0 (78 - 127) | 39.0 (17 - 54) | 39.0 (19 - 77) | 98.5 (78 - 123) | 32.5 (9 - 67) | 37.0 (6 - 68) |
| | **p-value*** | | | | | | <0.0001 | | | <0.0001 |
| t-PA (ng/mL) | **mean change (SD)** | 4.89 (2.118) | -0.92 (1.674) | -0.39 (1.756) | 5.06 (2.599) | -2.19 (2.395) | -2.18 (2.496) | 5.08 (2.395) | -1.77 (1.843) | -2.23 (1.819) |
| | **median change (min - max)** | 4.50 (2.0 - 10.6) | -0.75 (-4.4 - 2.4) | -0.30 (-5.5 - 3.4) | 4.30 (1.9 - 12.7) | -1.70 (-9.3 - 1.0) | -1.70 (-9.8 - 1.0) | 4.40 (1.5 - 13.1) | -1.40 (-5.9 - 1.4) | -1.65 (-6.1 - 1.2) |
| | **p-value*** | | | | | | 0.0046 | | | 0.0004 |
| PAI-1 (U/mL) | **mean change (SD)** | 1.41 (1.282) | - 0.12 (1.284) | 0.04 (1.152) | 1.29 (1.620) | -0.50 (1.783) | -0.68 (1.634) | 1.49 (1.484) | -0.64 (1.344) | -0.61 (1.671) |
| | **median change (min - max)** | 0.90 (0.5 - 5.5) | 0.00 (-3.5 - 2.8) | 0.10 (-2.6 - 2.1) | 0.50 (0.5 - 6.2) | 0.00 (-5.7 - 2.0) | 0.00 (-5.7 - 1.0) | 0.70 (0.5 - 6.2) | 0.00 (-5.5 - 0.9) | 0.00 (-5.2 - 3.3) |
| | **p-value*** | | | | | | 0.0751 | | | 0.0816 |
| ***Functional clotting tests*** | | | | | | | | | | |
| ETP-based APC resistance | **mean change (SD)** | 0.881 (0.2184) | 0.089 (0.2073) | 0.076 (0.2499) | 0.745 (0.2294) | 0.555 (0.2865) | 0.624 (0.3425) | 0.724 (0.1494) | 0.734 (0.2716) | 0.864 (0.3575) |
| | **median change (min - max)** | 0.830 (0.55 - 1.63) | 0.110 (-0.32 - 0.51) | 0.030 (-0.61 - 0.69) | 0.760 (0.33 - 1.47) | 0.500 (0.20 - 1.47) | 0.560 (0.06 - 1.55) | 0.705 (0.44 - 1.06) | 0.725 (0.36 - 1.37) | 0.820 (0.34 - 1.43) |
| | **p-value*** | | | | | | <0.0001 | | | <0.0001 |
| aPTT-based APC resistance | **mean change (SD)** | 0.49 (0.576) | 0.06 (0.666) | 0.08 (0.720) | 4.47 (1.264) | -0.00 (0.815) | 0.43 (0.816) | 5.08 (0.598) | -0.15 (0.704) | -0.02 (0.668) |
| | **median change (min - max)** | 4.90 (3.8 - 6.3) | 0.10 (-1.5 - 1.3) | 0.00 (-1.1 - 1.6) | 4.50 (1.4 - 6.9) | 0.20 (-2.1 - 1.4) | 0.10 (-0.5 - 2.4) | 5.00 (3.9 - 6.2) | -0.15 (-2.1 - 1.3) | -0.05 (-1.1 - 1.7) |
| aPTT-APC (sec) | **mean change (SD)** | 20.0 (1.40) | -0.1 (0.93) | -0.4 (1.03) | 19.9 (2.07) | -0.6 (0.84) | -0.3 (1.06) | 19.7 (1.63) | 0.2 (1.10) | 0.4 (1.16) |
| | **median change (min - max)** | 20.0 (17 - 23) | 0.0 (-2 - 2) | 0.0 (-3 - 1) | 20.0 (16 - 25) | -1.0 (-2 - 1) | -1.0 (-2 - 2) | 20.0 (17 - 23) | 0.0 (-2 - 2) | 1.0 (-2 - 2) |
| | **p-value*** | | | | | | 0.9836 | | | 0.0329 |
| aPTT+APC (sec) | **mean change (SD)** | 99.7 (16.98) | 0.4 (16.07) | -0.2 (15.98) | 89.4 (31.51) | -3.9 (19.07) | 6.4 (19.74) | 99.9 (17.22) | -1.8 (16.26) | 2.0 (15.45) |
| | **median change (min - max)** | 98.0 (69 - 145) | 0.0 (-39 - 29) | -1.0 (-26 - 37) | 86.0 (29 - 176) | 0.0 (-56 - 29) | 2.0 (-16 - 54) | 97.0 (67 - 130) | -0.5 (-50 - 31) | 2.0 (-22 - 35) |

| ***Markers of ongoing coagulation*** | | | | | | | | | | |
|---|---|---|---|---|---|---|---|---|---|---|
| | **mean change (SD)** | 0.1374 (0.0522) | 0.0082 (0.0387) | 0.0300 (0.0506) | 0.1265 (0.0520) | 0.0670 (0.0423) | 0.0948 (0.1272) | 0.1260 (0.0460) | 0.0696 (0.0527) | 0.0793 (0.0484) |

| **Table 1 - Haemostasis parameters** | | **E4/DRSP** | | | **EE/LNG** | | | **EE/DRSP** | | |
|---|---|---|---|---|---|---|---|---|---|---|
| **Mean (SD)** | | **Baseline** | **Change BL to Cycle 3** | **Change BL to Cycle 6** | **Baseline** | **Change BL to Cycle 3** | **Change BL to Cycle 6** | **Baseline** | **Change BL to Cycle 3** | **Change BL to Cycle 6** |
| **Median (min-max)** | | | | | | | | | | |
| Prothrombin Fragment 1+2 (nmol/L) | **median change (min - max)** | 0.1325 (0.060 - 0.261) | 0.0065 (-0.075 - 0.095) | 0.0290 (-0.064 - 0.173) | 0.1110 (0.060 - 0.332) | 0.0650 (0.003 - 0.165) | 0.0640 (-0.091 - 0.639) | 0.1135 (0.073 - 0.260) | 0.0500 (-0.016 - 0.201) | 0.0745 (0.001 - 0.208) |
| | **p-value*** | | | | | | 0.0021 | | | 0.0005 |
| D-dimer (µg/mL FEU) | **mean change (SD)** | 0.300 (0.0529) | 0.014 (0.1272) | 0.039 (0.0866) | 0.439 (0.4473) | -0.027 (0.1733) | -0.065 (0.4206) | 0.351 (0.1849) | 0.021 (0.1033) | 0.024 (0.0934) |
| | **median change (min - max)** | 0.270 (0.27 - 0.43) | 0.000 (-0.15 - 0.59) | 0.010 (-0.14 - 0.29) | 0.270 (0.27 - 2.28) | 0.000 (-0.57 - 0.16) | 0.020 (-1.92 - 0.29) | 0.270 (0.27-1.14) | 0.000 (-0.27 - 0.29) | 0.000 (-0.21 - 0.25) |

| ***Miscellaneous*** | | | | | | | | | | |
|---|---|---|---|---|---|---|---|---|---|---|
| SHBG (nmol/L) | **mean change (SD)** | 68.02 (21.137) | 37.71 (29.523) | 39.39 (31.290) | 73.16 (28.950) | 46.91 (35.855) | 47.41 (36.496) | 73.13 (23.086) | 183.27 (51.167) | 187.49 (59.528) |
| | **median change (min - max)** | 64.75 (25.3 - 117.9) | 30.30 (-21.3 - 102.7) | 30.30 (-20.4 - 108.0) | 67.30 (27.1 - 144.4) | 51.40 (-11.8 - 135.9) | 49.40 (-24.6 - 135.1) | 70.55 (36.2 - 125.6) | 173.95 (112.3 - 301.2) | 204.40 (89.3 - 335.4) |
| | **p-value*** | | | | | | 0.7045 | | | <0.0001 |
| Soluble E-Selectin (ng/mL) | **mean change (SD)** | 29.7 (11.827) | 0.90 (7.775) | 0.31 (6.857) | 37.10 (13.513) | -8.81 (6.114) | -11.02 (7.029) | 30.42 (10.822) | -6.24 (7.711) | -7.31 (5.150) |
| | **median change (min - max)** | 30.10 (11.6 - 58.9) | 2.20 (-16.0 - 27.0) | 0.95 (-10.4 - 30.4) | 37.90 (11.3 - 64.0) | -7.80 (-23.2 - 4.6) | -11.20 (-33.5 - 2.3) | 30.55 (13.8 - 63.4) | -6.75 (-26.2 - 14.7) | -6.85 (-22.1 - -0.1) |
| | **p-value*** | | | | | | <0.0001 | | | <0.0001 |
| APC. Activated protein C - TFPI. Free tissue factor pathway inhibitor - t-PA. Tissue type plasminogen inhibitor - PAI-1. Plasminogen activator inhibitor type 1 - SD. standard deviation - SHBG. Sex hormone binding globulin - vWF. von willebrand factor. | | | | | | | | | | |

| | | | | | | | | | | |
|---|---|---|---|---|---|---|---|---|---|---|
| *Additional exploratory non-parametric analysis was performed on the absolute change from baseline of the haemostatic, endocrine, liver protein and lipid profile parameters. To explore possible difference between the tratments with the regard to the change from baseline at cycle 6, the Kruskal-Wallis test was used. Where a possible difference was detected, pairwise comparisons of the treatments EE/LNG vs. E4/DRSP vs. E4/DRSP were done using the Dwass-Steel-Critchlow-Fligner procedure (without adjustments for multiplicity.) This test was applied on absolute change from baseline to Cycle 6. In such a case where a possible difference was detected, the corresponding p-value is indicated in the "p-value" line of the corresponding parameter, in the EE/LNG column for a comparison of EE/LNG vs. E4-DRSP and in the EE/DRSP column for a comparison of EE/DRSP vs. E4/DRSP. A p-value ≤ 0.05 indicates that the difference was statistically significant. | | | | | | | | | | |

**Table 2. Intraindividual relative change from baseline (hereinafter, "BL") to Cycles 3 and 6 (%, Mean and Median) (per-protocol population)**

| **Table 2 - Haemostasis parameters.** | | **E4/DRSP** | | **EE/LNG** | | **EE/DRSP** | |
|---|---|---|---|---|---|---|---|
| **Mean % (+/- SD)** | | **Change BL to** | **Change BL to** | **Change BL to** | **Change BL to** | **Change BL to** | **Change BL to** |
| Median % | | Cycle 3 | Cycle 6 | Cycle 3 | Cycle 6 | Cycle 3 | Cycle 6 |
| ***Coagulation factors*** | | | | | | | |
| Fibrinogen | **Mean** | 10.0 (21.6) | 9.8 (18.0) | 15.7 (28.8) | 13.7 (31.3) | 21.1 (24.9) | 18.6 (22.1) |
| | **Median** | 3.8 | 10.2 | 7.9 | 4.6 | 22.0 | 15.7 |
| Prothrombin activity | **Mean** | 6.2(8.2) | 6.3 (9.3) | 13.9 (9.2) | 11.3 (9.8) | 14.7 (9.4) | 11.0 (12.0) |
| | **Median** | 9.2 | 6.6 | 13.0 | 13.0 | 14.4 | 6.9 |
| Factor VII activity | **Mean** | -4.7 (10.3) | 0.5 (16.3) | -6.1 (16.5) | -6.9 (16.4) | 20.7 (19.3) | 18.8 (16.3) |
| | **Median** | -4.3 | -3.2 | -7.2 | -4.7 | 18.2 | 19.8 |
| Factor VIII activity | **Mean** | 10.1 (20.2) | 3.5 (22.8) | 10.2 (21.8) | 2.4 (27.8) | 19.2 (21.9) | 9.6 (25.2) |
| | **Median** | 11.3 | 4.9 | 8.6 | 2.9 | 20.6 | 8.6 |
| vWF | **Mean** | 6.3 (14.6) | 7.2 (18.6) | 0.5 (17.0) | -1.3 (18.6) | 11.6 (15.9) | 13.8 (18.1) |
| | **Median** | 5.1 | 4.8 | -1.9 | -2.1 | 7.9 | 13.4 |

| ***Anticoagulant proteins*** | | | | | | | |
|---|---|---|---|---|---|---|---|
| Antithrombin | **Mean** | 0.0 (5.6) | 0.3 (5.4) | -2.4 (6.3) | -3.7(6.7) | -1.8 (7.2) | -1.9 (8.6) |
| | **Median** | 0.0 | -0.9 | -2.1 | -5.0 | -2.3 | -3.5 |
| Protein S | **Mean** | -2.1 (13.5) | -2.1 (12.7) | 0.4 (17.8) | -5.7 (14.4) | -23.3 (8.9) | -29.2 (9.8) |
| | **Median** | 1.0 | -4.3 | -2.4 | -5.1 | -26.1 | -30.6 |
| Protein S. free | **Mean** | 4.5 (10.8) | 4.5 (11.6) | 3.8 (19.0) | -0.0 (15.6) | -21.7 (8.3) | -24.3 (11.9) |
| | **Median** | 7.8 | 4.9 | 2.5 | -3.4 | -21.1 | -22.2 |
| Protein C activity | **Mean** | 1.9 (10.2) | 2.0 (11.0) | 11.9 (12.8) | 10.7 (15.0) | 18.8 (12.5) | 19.4 (13.9) |
| | **Median** | 1.2 | 2.3 | 12.4 | 7.1 | 20 | 17.8 |
| TFPI | **Mean** | -4.7 (38.8) | -3.7 (24.2) | 5.7 (38.6) | -1.7 (30.2) | -3.3 (45.5) | -14.2 (23.3) |

| **Table 2 - Haemostasis parameters.** | | **E4/DRSP** | | **EE/LNG** | | **EE/DRSP** | |
|---|---|---|---|---|---|---|---|
| **Mean % (+/- SD)** | | **Change BL to** | **Change BL to** | **Change BL to** | **Change BL to** | **Change BL to** | **Change BL to** |
| **Median %** | | **Cycle 3** | **Cycle 6** | **Cycle 3** | **Cycle 6** | **Cycle 3** | **Cycle 6** |
| | **Median** | 9.0 | -8.4 | -2.6 | -6.2 | -18.9 | -20.4 |

| ***Fibrinolysis proteins*** | | | | | | | |
|---|---|---|---|---|---|---|---|
| Plasminogen | **Mean** | 11.4 (10.0) | 13.4 (10.0) | 41.3 (13.6) | 40.5 (14.4) | 37.0 (17.7) | 36.9 (14.9) |
| | **Median** | 11.9 | 12.2 | 45.3 | 40.4 | 32.0 | 35.3 |
| t-PA | **Mean** | -8.4 (0.3) | -0.0 (0.3) | -33.7 (25.9) | -34.5 (27.1) | -26.7 (33.7) | -35.6 (29.9) |
| | **Median** | -15.7 | -6.9 | -34.9 | -33.3 | -35.7 | -39.7 |
| PAI-1 | **Mean** | 46.1 (136.5) | 39.8 (92.9) | 17.0 (108.1) | -4.2 (67.4) | -16.9 (50.5) | -0.6 (80.6) |
| | **Median** | 0.0 | 20.0 | 0.0 | 0.0 | 0.0 | 0.0 |

| ***Functional clotting tests*** | | | | | | | |
|---|---|---|---|---|---|---|---|
| ETP-based APC resistance | **Mean** | 11.0 (22.5) | 9.9 (27.9) | 82.1 (49.8) | 92.0 (57.9) | 108.8 (49.5) | 125.1 (57.5) |
| | **Median** | 11.9 | 2.9 | 69.8 | 77.9 | 95.9 | 121.8 |
| aPTT-based APC resistance | **Mean** | 2.0(13.2) | 2.8(15.3) | 1.8(15.5) | 10.9(18.5) | -2.3 (13.3) | 1.1(13.5) |
| | **Median** | 2.1 | 0.0 | 4.5 | 5.0 | -3 | -1.0 |
| aPTT-APC | **Mean** | -0.4(4.6) | -1.7(4.9) | -3.0(4.2) | -1.1(5.5) | 1.4(5.6) | 2.0(5.9) |
| | **Median** | 0.0 | 0.0 | -4.2 | -4.0 | 0.0 | 4.8 |
| aPTT+APC | **Mean** | 1.8(15.5) | 1.3 (16.8) | -1.5 (16.8) | 9.7(23.0) | -0.7(15.8) | 3.2(16.0) |
| | **Median** | 0.0 | -1.1 | 0.0 | 4.5 | -0.4 | 2.5 |

| ***Markers of ongoing coagulation*** | | | | | | | |
|---|---|---|---|---|---|---|---|
| Prothrombin Fragment 1+2 | **Mean** | 9.6 (25.4) | 26.2 (37.5) | 58.8 (36.4) | 80.0 (73.7) | 57.6 (39.1) | 65.5 (38.4) |
| | **Median** | 6.7 | 23.1 | 62.2 | 71.1 | 47.6 | 64.3 |
| D-dimer | **Mean** | 7.8 (44.1) | 14.3 (27.8) | 4.7 (26.2) | 11.0 (42.7) | 6.4(25.9) | 11.5 (27.7) |
| | **Median** | 0 | 3.7 | 0.0 | 7.4 | 0.0 | 0.0 |

| **Table** 2 - **Haemostasis parameters.** | | **E4/DRSP** | | **EE/LNG** | | **EE/DRSP** | |
|---|---|---|---|---|---|---|---|
| **Mean % (+/- SD)** | | **Change BL to** | **Change BL to** | **Change BL to** | **Change BL to** | **Change BL to** | **Change BL to** |
| **Median %** | | **Cycle 3** | **Cycle 6** | **Cycle 3** | **Cycle 6** | **Cycle 3** | **Cycle 6** |
| ***Miscellaneous*** | | | | | | | |
| SHBG | **Mean** | 57.9 (40.67) | 61.3 (44.23) | 82.4 (76.92) | 80.0 (68.3) | 269.3 (105.3) | 274.7 (117.6) |
| | **Median** | 51.4 | 55.1 | 66.9 | 73.8 | 239.5 | 251.5 |
| **APC.** Activated protein C; TFPI. Free tissue factor pathway inhibitor; t-PA. Tissue type plasminogen inhibitor; PAI-1. Plasminogen activator inhibitor type 1; SD. standard deviation; SHBG. Sex hormone binding globulin; vWF. von willebrand factor. | | | | | | | |

**Table 3. Mean relative change from baseline to Cycle 3 and 6 (%) (per-protocol)**

| **Haemostasis parameters. mean (%)** | **E4/DRSP** | | **EE/LNG** | | **EE/DRSP** | |
|---|---|---|---|---|---|---|
| | Cycle 3 | Cycle 6 | Cycle 3 | Cycle 6 | Cycle 3 | Cycle 6 |
| ***Coagulation factors*** | | | | | | |
| Fibrinogen | 4.5 | 8.1 | 10.6 | 10.3 | 18.3 | 16.4 |
| Prothrombin activity | 6.0 | 5.9 | 13.2 | 11.2 | 14.3 | 10.4 |
| Factor VII activity | -5.3 | -1.4 | -7.7 | -8.8 | 20.9 | 18.5 |
| Factor VIII activity | 7.5 | 0.2 | 7.2 | 0.2 | 17.5 | 8.4 |
| vWF | 4.4 | 5.5 | -1.6 | -3.3 | 10.7 | 13.4 |

| ***Anticoagulant proteins*** | | | | | | |
|---|---|---|---|---|---|---|
| Antithrombin | -0.1 | 0.2 | --2.8 | -4.4 | -2.1 | -2.3 |
| Protein S | 0.6 | -2.5 | -1.2 | -7.0 | -23.7 | -29.6 |
| Protein S. free | 7.6 | 4.4 | 2.4 | -0.2 | -22.0 | -25.3 |
| Protein C activity | 1.6 | 1.7 | 11.0 | 9.7 | 18.1 | 18.7 |
| TFPI | -2.9 | -7.1 | 0.4 | -7.9 | -11.3 | -18.1 |

| ***Fibrinolysis proteins*** | | | | | | |
|---|---|---|---|---|---|---|
| Plasminogen | 10.9 | 13.1 | 40.1 | 40.7 | 35.8 | 36.1 |
| t-PA-18.8-8.0-43.2-42.9-34.8-42.5 | | | | | | |
| PAI-1 | -8.5 | 2.8 | -38.5 | -51.3 | -43.3 | -39.8 |

| ***Functional clotting tests*** | | | | | | |
|---|---|---|---|---|---|---|
| ETP-based APC resistance | 10.1 | 8.7 | 74.4 | 81.4 | 102.6 | 120.7 |
| aPTT-based APC resistance | 1.1 | 1.6 | -0.0 | 9.9 | -3.0 | -0.2 |
| aPTT-APC | -0.6 | -1.8 | -3.2 | -1.7 | -1.2 | 1.9 |
| aPTT+APC | -0.4 | -0.2 | -4.4 | 7.0 | -1.8 | 1.5 |

| ***Markers of ongoing coagulation*** | | | | | | |
|---|---|---|---|---|---|---|
| Prothrombin Fragment 1+2 | 5.9 | 21.8 | 53.0 | 74.8 | 55.2 | 61.0 |
| D-dimer | 4.7 | 12.9 | -6.1 | -13.6 | 6.1 | 7.7 |

| ***Miscellaneous*** | | | | | | |
|---|---|---|---|---|---|---|
| SHBG | 55.4 | 57.9 | 64.1 | 63.6 | 250.6 | 255.3 |
| APC. Activated protein C; TFPI. Free tissue factor pathway inhibitor; t-PA. Tissue type plasminogen inhibitor; PAI-1. Plasminogen activator inhibitor type 1; SHBG. Sex hormone binding globulin; vWF. von willebrand factor. | | | | | | |

### Calculation of the VTE risk

A clinical program parallel to the one reported above assessed the efficacy, cycle control, general safety and acceptability of E4/DRSP in healthy women aged 16-50 years and involved subject participation for 12 months (13 cycles, 1 cycle = 28 days). Women with a Body Mass index (BMI) up to 35.0 kg/m² were included in the study. In this clinical trial which involved 3417 subjects, a single case of VTE occurred. Based on the number of subjects in the trial and on the duration of COC administration for each subject, this occurrence can be converted into an estimated VTE risk for the COC of the invention of 3.7 VTE/10 000 women per year.

As reported in the Background Art section above, the estimated risk of blood clot occurrence in users of a CHC based on LNG, norethisterone or norgestimate is from 5 to 7 per 10,000 women during one year. For users of a CHC that contains drospirenone, the approximate risk of blood clot occurrence is from 9 to 12 per 10,000 women during one year.

It can thus be seen that the COC of the present invention compares favourably to earlier products of 2^{nd}, 3^{rd} and 4^{th} generation. For reference, the estimated risk of blood clot occurrence in non-CHC users who are not pregnant is around 2 per 10,000 women during one year (in more details, the article by de Bastos et al., "Combined oral contraceptives: venous thrombosis."; Cochrane Database Syst Rev. 2014 Mar 3;(3), reports incidences in cohorts of non-CHC users of 1.9 and 3.7 per 10,000 person years, together with earlier findings of 1.6 per 10,000 person years).

From the above it can be concluded that with a VTE occurrence of 3.7 per 10,000 women year, the COC of the present invention is at the upper limit of non-CHC users (3.7 per 10,000 women year reported in Lidegaard et al. (2011) BMJ 343:d6423 - see Table 2 on page 10 therein), and far apart from the lower limit of the safest generation of COCs of the prior art (risk of at least 5 per 10,000 women year reported for second generation COCs).

### Second determination of the ETP-based APC resistance

In an effort to confirm the data presented above, the present inventors have decided to run a second analysis of the plasma samples collected during the clinical trial described above, and in particular the measurement of the APC resistance based on ETP.

The laboratory protocol for analysing the plasma samples was improved and a new calibration routine was used. The following results were obtained after performing these new measurements, where Table 4 and Table 5 present results with the same formatting as Table 1 and Table 2 above, respectively.

**Table 4 Change from baseline (hereinafter, "BL") to Cycles 3 and 6 in levels of Activated Protein C Resistance - ETP based (per-protocol population)**

| **Table 4 APC-r (ETP-based)** | **E4/DRSP** | | | **EE/LNG** | | | **EE/DRSP** | | |
|---|---|---|---|---|---|---|---|---|---|
| | **Baseline** | **Change BL to Cycle 3** | **Change BL to Cycle 6** | **Baseline** | **Change BL to Cycle 3** | **Change BL to Cycle 6** | **Baseline** | **Change BL to Cycle 3** | **Change BL to Cycle 6** |
| **mean change +/- SD** | 1.803 (0.7770) | 0.618 (0.4889) | 0.472 (0.6360) | 1.496 (0.9739) | 2.229 (0.7831) | 2.042 (0.8805) | 1.375 (0.5951) | 2.970 (0.7936) | 3.014 (0.8506) |
| **median change (min** - **max)** | 1.665 (0.53 - 3.35) | 0.570 (-0.47 - 2.22) | 0.460 (-0.93 - 1.80) | 1.490 (0.00-5.03) | 2.250 (1.15 - 3.77) | 1.910 (0.17-3.43) | 1.370 (0.46-3.06) | 2.960 (1.64 - 4.53) | 3.055 (1.57 - 4.58) |
| **p-value*** | | | | | | < 0.0001 | | | < 0.0001 |

| | | | | | | | | | |
|---|---|---|---|---|---|---|---|---|---|
| ^{∗} Additional exploratory non-parametric analysis was performed on the absolute change from baseline. To explore possible difference between the treatments with regard to the change from baseline at cycle 6, the Kruskal-Wallis test was used. Where a possible difference was detected, pairwise comparisons of the treatments EE/LNG vs. E4/DRSP and EE/DRSP vs. E4/DRSP were done using the Dwass-Steel-Critchlow-Fligner procedure (without adjustment for multiplicity). In such a case, the corresponding p-value is indicated in the "p-value" line, in the EE/LNG column for a comparison of EE/LNG vs. E4/DRSP and in the EE/DRSP column for a comparison of EE/DRSP vs. E4/DRSP. A p-value ≤ 0.05 indicates that the difference was statistically significant. | | | | | | | | | |

**Table 5 Intraindividual relative change from baseline (hereinafter, "BL") to Cycles 3 and 6 (%, Mean and Median) (per-protocol population)**

| **Table 5 APC-r (ETP-based)** | **E4/DRSP** | | **EE/LNG** | | **EE/DRSP** | |
|---|---|---|---|---|---|---|
| | **Change BL to Cycle 3** | **Change BL to Cycle 6** | **Change BL to Cycle 3** | **Change BL to Cycle 6** | **Change BL to Cycle 3** | **Change BL to Cycle 6** |
| **Mean % (+/- SD)** | 42.0 (34.6) | 36.7 (52.6) | 182.7 (114.3) | 165.8 (98.6) | 266.3 (162.1) | 268.1 (170.7) |
| **Median % (min - max)** | 39.5 (-19.0 - 117.0) | 30.0 (-53.0 - 233.0) | 165.0 (33.0 - 496.0) | 164.5 (30.0 - 424.0) | 229.0 (91.0 - 781.0) | 218.5 (99.0 - 763.0) |

Despite the protocol modifications and the full rerun of all plasma samples, the trend reported in Tables 1-3 above for the ETP-based APC resistance is fully confirmed in Tables 4 and 5 above. In particular, the advantages of the COC of the present invention are directly apparent by comparison with a 4^{th} generation COC (Yaz^{®}), but also, and more importantly, a 2^{nd} generation COC (Melleva^{®}).

Statistical analyses additionally reveal very significant differences from baseline, and especially so for the Yaz^{®} and Melleva^{®} comparator COCs. More importantly, as shown by p-values displayed in Tables 1 and 4, there were very significant differences between the COC of the invention and both comparators, while both comparators were less different to each other.

From the above it appears that the COC of the invention is significantly different from prior art 2^{nd} and 4^{th} generation COCs. Altogether, the measured parameters demonstrate that the haemostatic profile of the COC of the invention is at least comparable to a safest 2^{nd} generation LNG-containing COC, and shows a more favourable effect on haemostasis parameters than a 4^{th} generation DRSP-containing COC.

## Claims

1. A composition comprising from 10 to 25 mg estetrol and from 1 to 4 mg drospirenone, for use in oral contraception, wherein administration of said composition does not result in hemostatic changes exceeding the boundaries of the normal hemostatic range.

2. The composition for use according to claim 1, wherein said use results in reduced cardiovascular effects in said subject.

3. The composition for use according to claim 1, wherein said use results in preventing risk increase of developing venous thromboembolism (VTE) and/or of developing aortic thromboembolism (ATE) in a subject compared to other combined oral contraceptives (COCs).

4. A method of avoiding hemostatic changes exceeding the boundaries of the normal hemostatic range in subjects using a combined oral contraceptive, comprising administering a combined oral contraceptive comprising from 10 to 25 mg estetrol and from 1 to 4 mg drospirenone.

5. A method of oral contraception comprising administering to said subject a composition comprising from 10 to 25 mg estetrol and from 1 to 4 mg drospirenone, wherein administration of said composition does not result in hemostatic changes exceeding the boundaries of the normal hemostatic range.

6. The method according to claim 4 or 5, resulting in preventing risk increase of developing venous thromboembolism (VTE) and/or of developing aortic thromboembolism (ATE) associated with the use of COC's in subjects using a combined oral contraceptive.

7. The method according to any one of claims 4 to 6, resulting in preventing risk increase of developing VTE in subjects using a combined oral contraceptive.

8. The composition for use or the method according to any one of claims 1 to 7, wherein the normal hemostatic range is defined as the prediction interval that 95% of the population fall into.

9. The composition for use or the method according to any one of claims 1 to 8, wherein said hemostatic change is determined based on any one or more of the following markers: SHBG, free TFPI, free and total Protein-S, Protein-S activity, CBG, Ceruloplasmin, antithrombin III, APC resistance, protein-C activity, D-dimer, Prothrombin, Prothrombin fragment 1+2, Factor VII, Factor VIII, von Willebrand factor, Factor II, PAI-I, t-PA, plasminogen, E-selectin and fibrinogen.

10. The composition for use or the method according to any one of claims 1 to 9, wherein said hemostatic change is determined based on a change in level of Protein-S or TFPI when compared to the normal hemostatic range.

11. The composition for use or the method according to any one of claims 1 to 10, wherein said hemostatic change is measured after one cycle of treatment, after two cycles of treatment or after three cycles of treatment.

12. The composition for use or the method according to any one of claims 1 to 11, comprising from 15 to 20 mg estetrol.

13. The composition for use or the method according to any one of claims 1 to 12, comprising about 15 mg estetrol.

14. The composition for use or the method according to any one of claims 1 to 12, comprising about 20 mg estetrol.

15. The composition for use or the method according to any one of claims 1 to 14, comprising from about 3 mg drospirenone.

16. The composition for use or the method according to any one of claims 1 to 15, wherein said estetrol is estetrol monohydrate.

17. The composition for use or the method according to any one of claims 1 to 16, wherein said composition is administered orally.

18. The composition for use or the method according to any one of claims 1 to 17, wherein said composition is administered during at least 21 days, or during 24 days, respectively followed by an administration-free period of about 7 to 4 days.
